# EUROPÄISCHE PATENTSCHRIFT

(11) **EP 2 323 621 B1**
(45) Veröffentlichungstag und Bekanntmachung des Hinweises auf die Patenterteilung: **05.10.2016**
(21) Anmeldenummer: 09780877.8
(22) Anmeldetag: 21.07.2009
(51) Int. Cl.: A61K 8/81, A61Q 5/06, A61K 8/73, A61K 8/04

(54) **MITTEL FÜR KERATINHALTIGE FASERN, ENTHALTEND MINDESTENS EIN SPEZIELLES AMPHIPHILES KATIONISCHES POLYMER, MINDESTENS EIN DAVON VERSCHIEDENES KATIONISCHE STYLINGPOLYMER UND MINDESTENS EIN FILMBILDENDES NICHTIONISCHES UND/ODER FESTIGENDES NICHTIONISCHES POLYMER**
AGENT FOR FIBRES CONTAINING KERATIN, COMPRISING AT LEAST ONE SPECIFIC AMPHIPHILIC CATIONIC POLYMER, AT LEAST ONE CATIONIC STYLING POLYMER THAT IS DIFFERENT THEREFROM AND AT LEAST ONE FILM-FORMING NON-IONIC AND/OR STABILIZING NON-IONIC POLYMER
PRÉPARATION POUR FIBRES KÉRATINIQUES CONTENANT AU MOINS UN POLYMÈRE CATIONIQUE AMPHIPHILE SPÉCIAL, AU MOINS UN POLYMÈRE DE COIFFAGE CATIONIQUE DIFFÉRENT DU PREMIER POLYMÈRE, ET AU MOINS UN POLYMÈRE NON IONIQUE FILMOGÈNE ET/OU NON IONIQUE FIXANT

(30) Priorität: 18.08.2008 DE 102008038110; 28.11.2008 DE 102008059480
(43) Veröffentlichungstag der Anmeldung: 25.05.2011
(73) Patentinhaber: Henkel AG & Co. KGaA, 40589 Düsseldorf (DE)
(72) Erfinder: KAFTAN, Pamela, 22525 Hamburg (DE); MÜLLER, Burkhard, 21075 Hamburg (DE)
(86) Internationale Anmeldenummer: PCT/EP2009/059357
(87) Internationale Veröffentlichungsnummer: WO 2010/020505

(56) Entgegenhaltungen:
- WO-A1-2006/050788
- DE-A1- 19 937 434
- DE-U1- 20 023 230
- US-B1- 6 852 815
- RAYMOND RIGOLETTO ET AL: "Polyquaternium-69: A New Fixative Polymer with Enhanced Styling Benefits" INTERNET CITATION 1. Januar 2007 (2007-01-01), XP007910768 Gefunden im Internet: URL:http://www.cosmeticsciencetechnology.c om/articles/samples/1281.pdf> [gefunden am 2009-12-03]
- ISP: "AquaStyle 300" INTERNET CITATION 6. Dezember 2006 (2006-12-06), Seiten 1-94, XP007910767 Gefunden im Internet: URL:http://www.ispjapan.co.jp/pc_refguide/ pdf/AquaStyle_300_jp.pdf> [gefunden am 2009-12-03]

## Beschreibung

Die vorliegende Erfindung betrifft Mittel zur temporären Haarverformung enthaltend eine Kombination aus mindestens einem speziellen amphiphilen kationischen Polymer mit mindestens einem davon verschiedenen, kationischen Stylingpolymer und mindestens einem filmbildenden nichtionischen und/oder festigenden nichtionischen Polymer, die Verwendung dieser Mittel zur temporären Verformung keratinhaitiger Fasern und Aerosolhaarsprays/-schäume auf Basis dieser Mittel.

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Stylingmittel zu entwickeln, die alle gewünschten Eigenschaften in Kombination aufweisen, bereitet nach wie vor Schwierigkeiten. Insbesondere gilt dies für die Kombination von starkem Halt einerseits und einfacher, gleichmäßiger Applikation auf die keratinhaltigen Fasern andererseits.

Die DE-A-199 37 434 offenbart ein Haarbehandlungsmittel in Form einer Sprühlotion enthaltend
a) 0,01 bis 20 Gew.- % eines Terpolymers aus Vinylpyrrolidon, Vinylcaprolactam und einem basischen Acrylamidmonomer,
b) 0,01 bis 10 Gew.- % eines weiteren, haarpflegenden, kationischen Polymers und
c) 0,01 bis 15 Gew.- % eines filmbildenden, nichtionischen Polymers.

Der Einsatz von Polyquaternium-69 als Haarfixierungspolymer mit geringer Schuppenbildung, sehr guter Festigkeits- und Gelwirkung wird in der Veröffentlichung "Polyquaternium-69: A New Fixative Polymer with Enhanced Styling Benefits" offenbart (Gefunden im Internet http://www.cosmeticsciencetechnology.com/articles/samples/1281.pdf).

Aufgabe der vorliegenden Erfindung war es daher, ein Mittel zur temporären Verformung und/oder zur Pflege keratinischer Fasern zur Verfügung zu stellen, das sich durch einen hohen Haltegrad bzw. durch eine hohe Pflegewirkung auszeichnet und insbesondere eine hervorragende Handhabbarkeit während der Applikation auf die keratinhaltigen Fasern besitzt.

Es wurde nunmehr überraschenderweise gefunden, dass dies durch eine Kombination spezieller Polymere erreicht werden kant.

Ein erster Gegenstand der vorliegenden Erfindung ist daher ein Mittel zur temporären Haarverformung keratinhaltiger Fasern, insbesondere menschlichem Haar, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 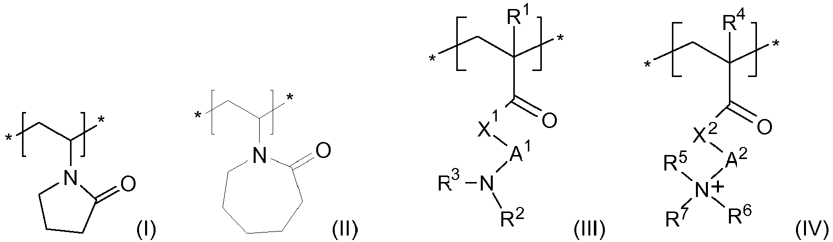 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe und
(b) mindestens ein weiteres filmbildendes kationisches und/oder festigendes kationisches Polymer und
(c) mindestens ein filmbildendes nichtionisches und/oder festigendes nichtionisches Polymer.

Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Festigende Polymere tragen zum Halt und/oder zum Aufbau des Haarvolumens und der Haarfülle der Gesamtfrisur bei. Diese Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Als eine Testmethode für die festigende Wirkung eines Polymers wird häufig der so genannte curl-retention - Test angewendet.

Gemäß obiger Formeln und allen folgenden Formeln steht eine chemische Bindung, die mit dem Symbol * gekennzeichnet ist, für eine freie Valenz des entsprechenden Strukturfragments.

Zur Kompensation der positiven Polymerladung in dem erfindungsgemäß Mittel dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Beispiele für erfindungsgemäße (C₁ bis C₄)-Alkylgruppen sind Methyl, Ethyl, Propyl, Isopropyl, Butyl, Sec-Butyl, Isobutyl, tert-Butyl.
Beispiele für erfindungsgemäße (C₈ bis C₃₀)-Alkylgruppen sind Octyl (Capryl), Decyl (Caprinyl), Dodecyl (Lauryl), Tetradecyl (Myristyl), Hexadecyl (Cetyl), Octadecyl (Stearyl), Eicosyl (Arachyl), Docosyl (Behenyl).

Die erfindungsgemäßen amphiphilen, kationischen Polymere weisen bevorzugt ein Molekulargewicht von 10000 g/mol bis 50000000 g/mol, insbesondere von 50000 g/mol bis 5000000 g/mol, besonders bevorzugt von 75000 g/mol bis 1000000 g/mol, auf.

Im Sinne der Erfindung bevorzugte Mittel enthalten die amphiphilen, kationischen Polymere (a) in einer Menge von 0,1 Gew.-% bis 20,0 Gew.-%, besonders bevorzugt von 0,2 Gew.-% bis 10,0 Gew.-%, ganz besonders bevorzugt von 0,5 bis 5,0 Gew.%, jeweils bezogen auf das Gewicht des Mittels.

Besonders vorteilhaft erweisen sich die Eigenschaften des erfindungsgemäßen Mittels, wenn es als Aerosolspray, Aerosolschaum, Pumpspray oder Pumpschaum konfektioniert wird. Diese bevorzugte Form der Konfektionierung wird später im Detail beschrieben.

Nachfolgende amphiphile, kationische Polymere (a) finden erfindungsgemäß bevorzugt in den erfindungsgemäßen Mitteln Einsatz, wenn die amphiphilen, kationischen Polymere (a) bezüglich oben genannter Formeln (I) bis (IV) eines oder mehrere der folgenden Merkmale erfüllen:
- R¹ und R⁴ bedeuten jeweils eine Methylgruppe,
- X¹ steht für eine Gruppe NH,
- X² steht für eine Gruppe NH,
- X¹ und X² stehen für eine Gruppe NH,
- A¹ und A² stehen unabhängig voneinander für Ethan-1,2-diyl oder Propan-1,3-diyl,
- R², R³, R⁵ und R⁶ stehen unabhängig voneinander für Methyl oder Ethyl, (besonders bevorzugt für Methyl),
- R⁷ steht für eine (C₁₀ bis C₂₄)-Alkylgruppe, insbesondere für Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

Es ist erfindungsgemäß bevorzugt, die Struktureinheit der Formel (III) aus aus mindestens einer Struktureinheit der Formel (III-1) bis (III-8) auszuwählen

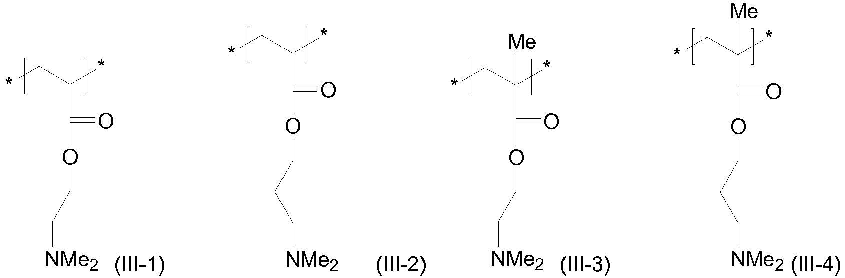

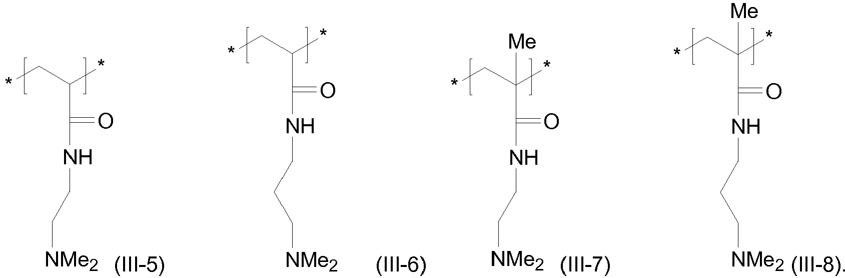

Ausserdem erweist es sich als besonders bevorzugt, als Struktureinheit der Formel (III) die Struktureinheit gemäß Formel (III-7) und/oder der Formel (III-8) zu wählen. Die Struktureinheit der Formel (III-8) ist erfindungsgemäß eine ganz besonders bevorzugte Struktureinheit.

Ferner stellte es sich mit Blick auf die Lösung der Aufgabe als bevorzugt heraus, wenn die Struktureinheit der Formel (IV) ausgewählt wird, aus mindestens einer Struktureinheit der Formeln (IV-1) bis (IV-8) 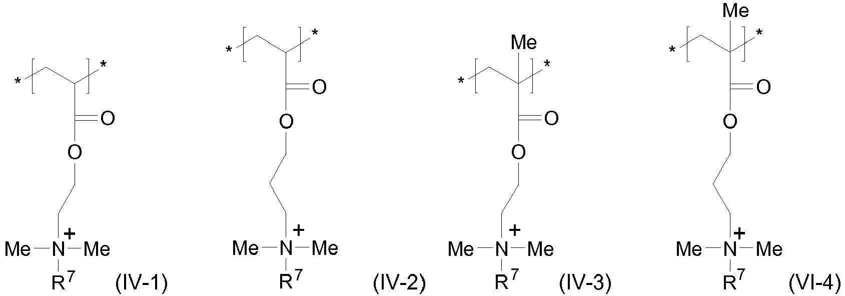 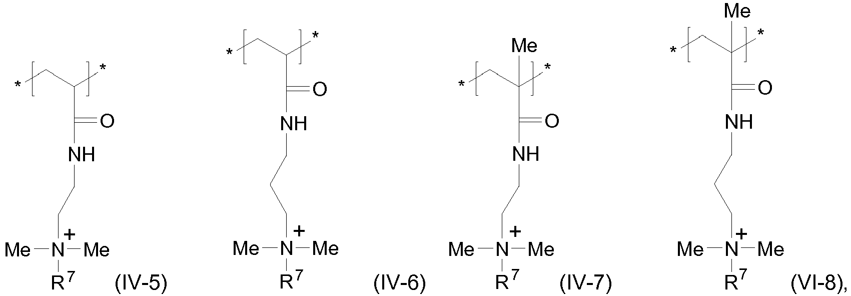 worin R⁷ jeweils für eine (C₈ bis C₃₀)-Alkylgruppe steht.

Als wiederum besonders bevorzugte Struktureinheit der Formel (IV) gelten die Struktureinheiten der Formel (IV-7) und/oder der Formel (IV-8), worin jeweils R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl). Die Struktureinheit der Formel (IV-8) stellt erfindungsgemäß eine ganz besonders bevorzugte Struktureinheit der Formel (IV) dar.

Ein ganz besonders bevorzugt in dem erfindungsgemäßen Mittel enthaltenes amphiphiles, kationisches Polymer umfasst mindestens eine Struktureinheit der Formel (I), mindestens eine Struktureinheit der Formel (II), mindestens eine Struktureinheit der Formel (III-8) und mindestens eine Struktureinheit der Formel (IV-8), 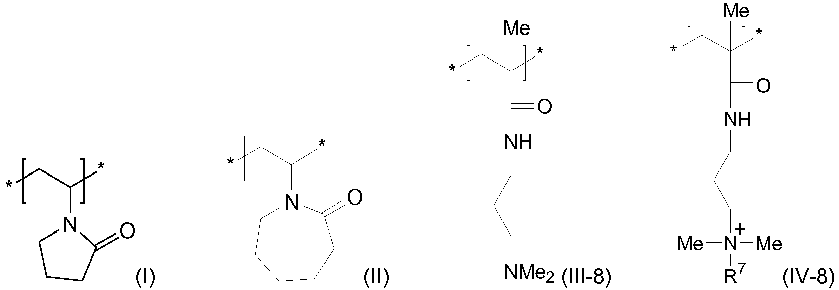 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

Ein ganz besonders bevorzugtes erfindungsgemäßes amphiphiles, kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch, Molekulargewicht 350000) von der Firma ISP vertrieben wird.

Zusätzlich enthält das erfindungsgemäße Mittel zwingend mindestens ein weiteres filmbildendes kationisches und/oder festigendes Polymer. Diese Polymere sind von den amphiphilen, kationischen Polymeren (a) verschieden.

Die zusätzlichen filmbildenden kationischen und/oder festigenden kationischen Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.

Im Sinne der Erfindung bevorzugte Mittel enthalten die filmbildenden kationischen und/oder festigenden kationischen Polymere (b) in einer Menge von 0,1 Gew.-% bis 20,0 Gew.-%, besonders bevorzugt von 0,2 Gew.-% bis 10,0 Gew.-%, ganz besonders bevorzugt von 0,5 Gew.-% bis 5,0 Gew.-%, jeweils bezogen auf das Gewicht des Mittels.

Die kationischen filmbildenden und/oder kationischen festigenden Polymere können erfindungsgemäß aus kationischen, quaternisierten Cellulose-Derivaten ausgewählt werden.

Im Rahmen dieser Ausführungsform eignen sich solche Mittel, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 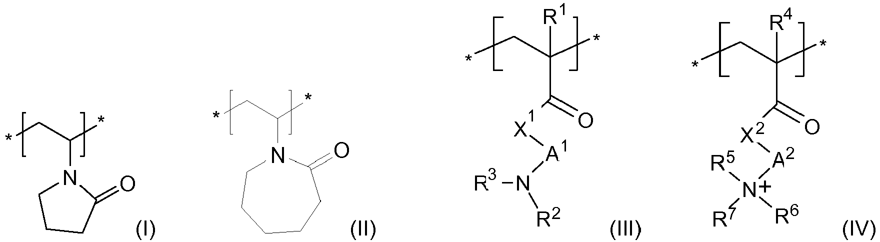 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe und
(b) mindestens ein zusätzliches filmbildendes kationisches und/oder festigendes kationisches Polymer ausgewählt aus der Gruppe der kationischen, quaternisierten Cellulosederivate und
(c) mindestens ein filmbildendes nichtionisches und/oder festigendes nichtionisches Polymer.

Es gelten wiederum insbesondere solche Mittel im Rahmen dieser Ausführungsform als geeignet, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 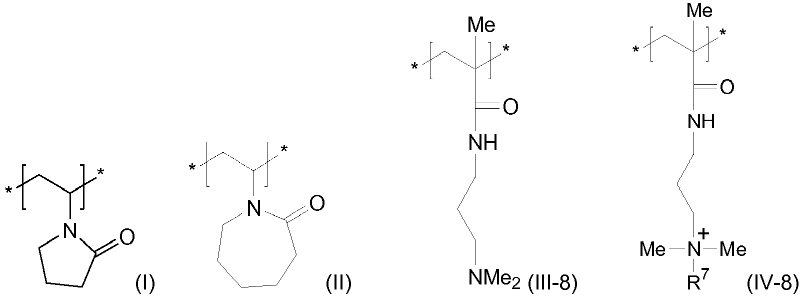 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer ausgewählt aus der Gruppe der kationischen, quaternisierten Cellulosederivate, und
(c) mindestens ein filmbildendes nichtionisches und/oder festigendes nichtionisches Polymer.

Es erweisen sich generell solche kationischen, quaternisierten Cellulosen als im Sinne der Erfindung vorteilhaft, die in einer Seitenkette mehr als eine permanente kationische Ladung tragen. Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet, welche beispielsweise unter den Bezeichnungen Celquat^{®} H 100, Celquat^{®} L 200 von der Firma National Starch vertrieben werden.

Daher stellen im Rahmen dieser Ausführungsform erfindungsgemäße Mittel als geeignet heraus, welche in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8) 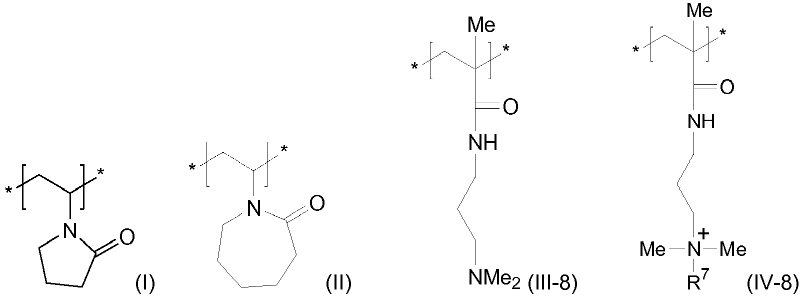 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer ausgewählt aus kationischen, quaternisierten Cellulosederivaten, die durch Reaktion von Hydroxyethylcellulose mit einem Dimethyldiallylammonium-Reaktanden (insbesondere Dimethyldiallylammoniumchlorid) - gegebenenfalls in Gegenwart weiterer Reaktanden - hergestellt werden
   und
(c) mindestens ein filmbildendes nichtionisches und/oder festigendes nichtionisches Polymer.

Im Rahmen dieser vorgenannten Ausführungsformen eignen sich die zuvor genannten bevorzugten Ausführungsformen des amphiphilen, kationischen Polymers (a) als geeignet (*vide supra*). Gleichsam gelten alle zuvor genannten bevorzugten Mengenangaben bezüglich der Polymerkomponenten (a) und (b) des erfindungsgemäßen Mittels auch für diese Ausführungsformen *mutatis mutandis* als gut geeignet.

Weiterhin eignen sich solche kationischen filmbildenden und/oder kationischen festigenden Polymere (b), die mindestens eine Struktureinheit der Formel (I) und mindestens eine Struktureinheit der Formel (VI) und gegebenenfalls mindestens eine Struktureinheit der Formel (V) umfassen 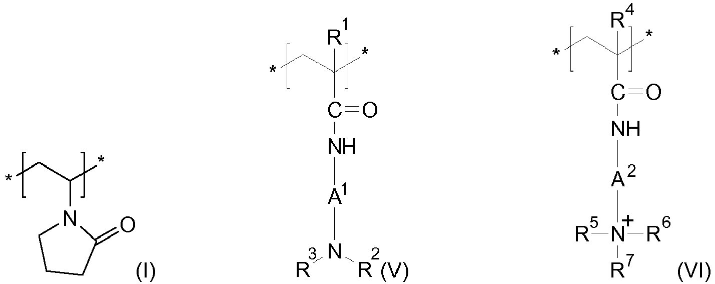 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

Zur Kompensation der positiven Ladung des Monomers (VI) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Als erfindungsgemäß geeignet gelten daher im Sinne der vorliegenden Erfindung solche Mittel, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 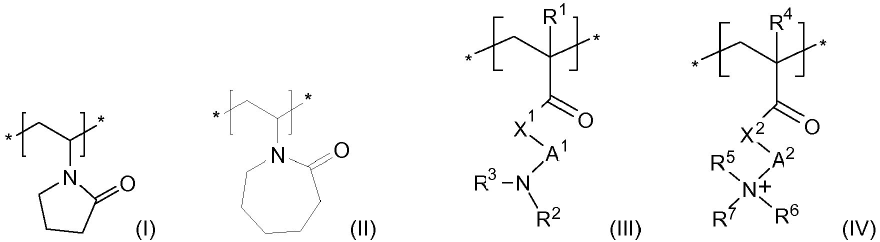 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 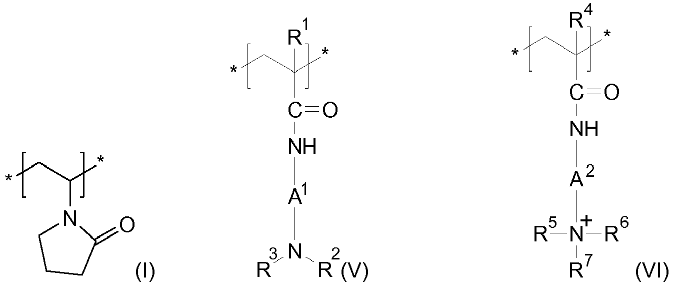 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) mindestens ein filmbildendes nichtionisches und/oder festigendes nichtionisches Polymer.

Es gelten weiterhin insbesondere solche Mittel im Rahmen dieser Ausführungsform als erfindungsgemäß geeignet, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8) 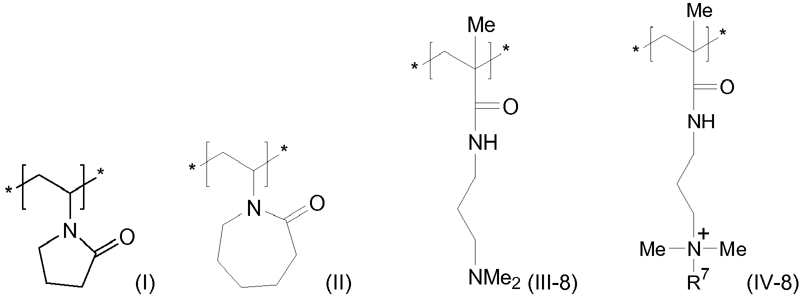
   worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
      und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 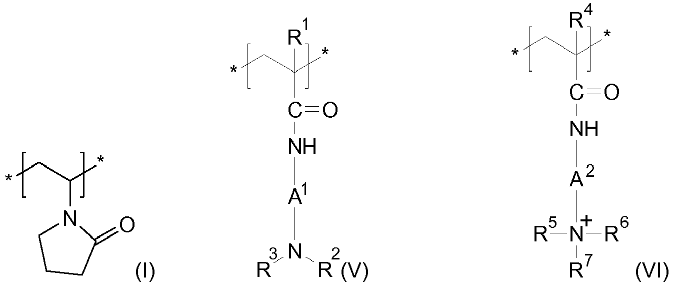 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) mindestens ein filmbildendes nichtionisches und/oder festigendes nichtionisches Polymer.

Zur Kompensation der positiven Ladung des Monomers (VI) gilt jeweils das oben gesagte.

Geeignete Verbindungen sind beispielsweise als
- Copolymere aus mit Diethylsulfat quaterniertem Dimethylaminoethylmethacrylat, mit Vinylpyrrolidon mit der INCI-Bezeichnung Polyquaternium-11 unter den Bezeichnungen Gafquat^{®} 440, Gafquat^{®}734, Gafquat^{®}755 (jeweils Firma ISP) sowie Luviquat PQ 11 PN (Firma BASF SE),
- Copolymere aus Methacryloylaminopropyllauryldimethylammonium chlorid mit Vinylpyrrolidon und Dimethylaminopropylmethacrylamid mit der INCI-Bezeichnung Polyquaternium-55 unter den Handelsnamen, Styleze^{®} W-10, Styleze^{®} W-20 (Firma ISP),
im Handel erhältlich.

Im Rahmen dieser Ausführungsformen eignen sich die zuvor genannten bevorzugten Ausführungsformen des amphiphilen, kationischen Polymers (a) als bevorzugt (*vide supra*). Gleichsam gelten alle zuvor genannten bevorzugten Mengenangaben bezüglich der Polymerkomponenten (a) und (b) des erfindungsgemäßen Mittels auch für diese Ausführungsformen *mutatis mutandis* als bevorzugt.

Als im Sinne der Erfindung besonders bevorzugt verwendbare filmbildende und/oder festigende Polymere ausgewählt aus kationischen Polymeren die mindestens eine Struktureinheit enthalten, die ein permanent kationisiertes Stickstoffatom aufweisen, dienen weiterhin solche kationischen filmbildenden und/oder kationischen festigenden Copolymere (b), die mindestens ein Strukturelement der Formel (M1) enthalten 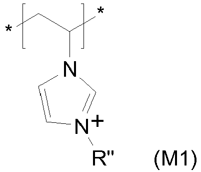 worin
R für eine (C₁ bis C₄)-Alkylgruppe, insbesondere eine Methylgruppe, steht, und zusätzlich mindestens ein weiteres kationisches und/oder nichtionisches Strukturelement aufweisen.

Als besonders bevorzugt gelten daher im Sinne der vorliegenden Erfindung solche Mittel, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 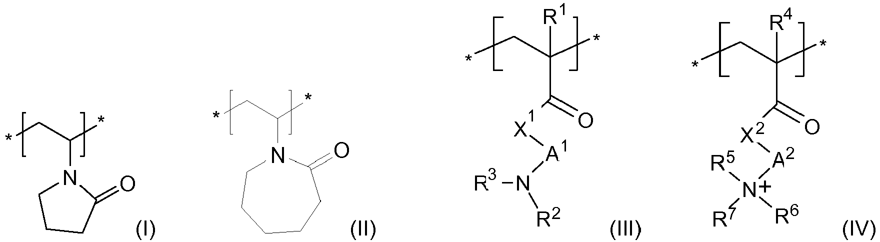 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer, das als mindestens eine Struktureinheit mit einem permanent kationisierten Stickstoffatom mindestens ein Strukturelement der Formel (M1) enthält 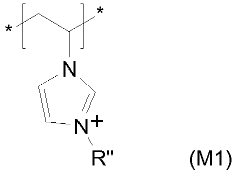 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) mindestens ein filmbildendes nichtionisches und/oder festigendes nichtionisches Polymer.

Zur Kompensation der positiven Polymerladung der Komponente (b) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Es gelten weiterhin insbesondere solche Mittel im Rahmen dieser Ausführungsform als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8) 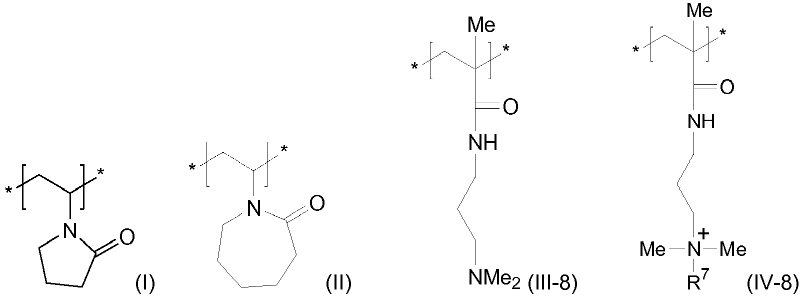 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) enthält 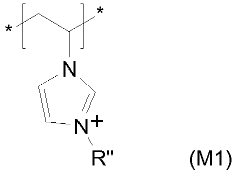 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) mindestens ein filmbildendes nichtionisches und/oder festigendes nichtionisches Polymer.

Insbesondere eignet sich als amphiphiles kationisches Polymer (a) besonders bevorzugt das zuvor erwähnte Polymer mit der INCI-Bezeichung Polyquaternium 69 (*vide supra*).

Zur Kompensation der positiven Polymerladung der Komponente (b) gilt das oben gesagte.

Es ist wiederum erfindungsgemäß bevorzugt, wenn in dem erfindungsgemäßen Mittel neben dem amphiphilen kationischen Polymer (a) als kationisches filmbildendes und/oder kationisches festigendes Polymer (b) dieser Ausführungsform, mindestens ein Copolymer (b1) enthalten ist, das neben mindestens einem Strukturelement der Formel (M1) zusätzlich ein Strukturelement der Formel (I) umfasst 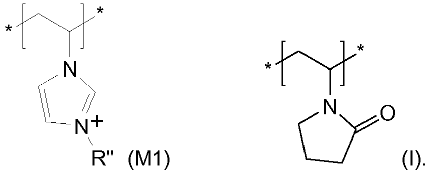 worin
R für eine (C₁ bis C₄)-Alkylgruppe, insbesondere eine Methylgruppe, steht.

Zur Kompensation der positiven Polymerladung der Copolymere (b1) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Ganz besonders bevorzugte kationische filmbildende und/oder kationische festigende Polymere als Copolymere (b1) enthalten 10 bis 30 Mol-%, vorzugsweise 15 bis 25 Mol.-% und insbesondere 20 Mol.-% Struktureinheiten gemäß Formel (M1) und 70 bis 90 Mol.-%, vorzugsweise 75 bis 85 Mol.-% und insbesondere 80 Mol.-% Struktureinheiten gemäß Formel (I).

Hierbei ist besonders bevorzugt, wenn die Copolymere (b1) neben Polymereinheiten, die aus dem Einbau der genannten Struktureinheiten gemäß Formel (M1) und (I) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b1) ausschließlich aus Struktureinheiten der Formel (M1) mit R" = Methyl und (I) aufgebaut und lassen sich durch die allgemeine Formel (Poly1) 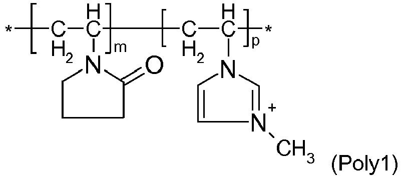 beschreiben, wobei die Indices m und p je nach Molmasse des Polymers variieren und nicht bedeuten sollen, daß es sich um Blockcopolymere handelt. Vielmehr können Struktureinheiten der Formel (M1) und der Formel (I) im Molekül statistisch verteilt vorliegen.

Wird in zur Kompensation der positiven Ladung des Polymers der Formel (Poly1) ein Chloridion verwendet, so werden diese N-Methylvinylimidazol/Vinylpyrrolidon-Copolymere werden laut INCI-Nomenklatur als Polyquaternium-16 bezeichnet und sind beispielsweise von der BASF unter den Handelsnamen Luviquat^{®} Style, Luviquat^{®} FC 370, Luviquat^{®} FC 550, Luviquat^{®} FC 905 und Luviquat^{®} HM 552

Wird in zur Kompensation der positiven Ladung des Polymers der Formel (Poly1) ein Methosulfat verwendet, so werden diese N-Methylvinylimidazol/Vinylpyrrolidon-Copolymere werden laut INCI-Nomenklatur als Polyquaternium-44 bezeichnet und sind beispielsweise von der BASF unter den Handelsnamen Luviquat^{®} UltraCare erhältlich.

Besonders bevorzugte erfindungsgemäße Mittel enthalten ein Copolymer (b1), insbesondere der Formel (Poly1), das Molmassen innerhalb eines bestimmten Bereiches aufweist. Hier sind erfindungsgemäße Mittel bevorzugt, bei denen das Copolymer (b1) eine Molmasse von 50 bis 400 kDa, vorzugsweise von 100 bis 300 kDa, weiter bevorzugt von 150 bis 250 kDa und insbesondere von 190 bis 210 kDa aufweist.

Zusätzlich zu dem bzw. den Copolymer(en) (b1) oder an dessen bzw. deren Stelle können die erfindungsgemäßen Mittel auch Copolymere (b2) enthalten, die ausgehend vom Copolymer (b1) als zusätzliche Struktureinheiten Struktureinheiten der Formel (II) aufweisen

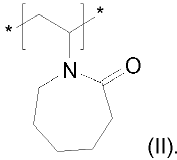

Weitere besonders bevorzugte erfindungsgemäße Mittel sind somit dadurch gekennzeichnet, daß sie als kationisches filmbildendes und/oder kationisches festigendes Polymer (b) mindestens ein Copolymer (b2) enthalten, das mindestens eine Struktureinheit gemäß Formel (M1-a) und mindestens eine Struktureinheit gemäß Formel (I) und mindestens weitere Struktureinheit gemäß Formel (II) enthält

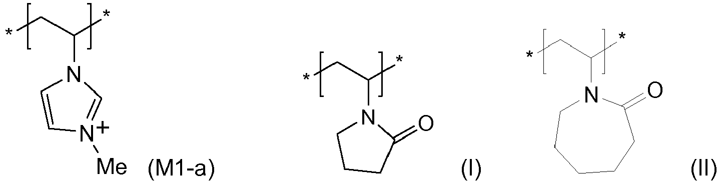

Auch hierbei ist besonders bevorzugt, wenn die Copolymere (b2) neben Polymereinheiten, die aus dem Einbau der genannten Struktureinheiten gemäß Formel (M1-a), (I) und (II) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b2) ausschließlich aus Struktureinheiten der Formeln (M1-a), (I) und (II) aufgebaut und lassen sich durch die allgemeine Formel (Poly2) 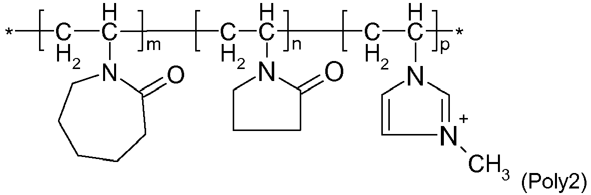 beschreiben, wobei die Indices m, n und p je nach Molmasse des Polymers variieren und nicht bedeuten sollen, daß es sich um Blockcopolymere handelt. Vielmehr können Struktureinheiten der besagten Formeln im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly2) ein Methosulfat verwendet werden solche N-Methylvinylimidazol/Vinylpyrrolidon/Vinylcaprolactam-Copolymere laut INCI-Nomenklatur als Polyquarternium-46 bezeichnet und sind beispielsweise von der BASF unter dem Handelsnamen Luviquat^{®} Hold erhältlich.

Ganz besonders bevorzugte Copolymere (b2) enthalten 1 bis 20 Mol-%, vorzugsweise 5 bis 15 Mol.-% und insbesondere 10 Mol.-% Struktureinheiten gemäß Formel (M1-a) und 30 bis 50 Mol.-%, vorzugsweise 35 bis 45 Mol.-% und insbesondere 40 Mol.-% Struktureinheiten gemäß Formel (I) und 40 bis 60 Mol.-%, vorzugsweise 45 bis 55 Mol.-% und insbesondere 60 Mol.-% Struktureinheiten gemäß Formel (II).

Besonders bevorzugte erfindungsgemäße Mittel enthalten ein Copolymer (b2), das Molmassen innerhalb eines bestimmten Bereiches aufweist. Hier sind erfindungsgemäße Mittel bevorzugt, bei denen das Copolymer (b2) eine Molmasse von 100 bis 1000 kDa, vorzugsweise von 250 bis 900 kDa, weiter bevorzugt von 500 bis 850 kDa und insbesondere von 650 bis 710 kDa aufweist.

Zusätzlich zu dem bzw. den Copolymer(en) (b1) und/oder (b2) oder an dessen bzw. deren Stelle können die erfindungsgemäßen Mittel als filmbildendes kationische und/oder festigendes kationisches Polymer (b) auch Copolymere (b3) enthalten, die als Struktureinheiten Struktureinheiten der Formeln (M1-a) und (I) aufweisen, sowie weitere Struktureinheiten aus der Gruppe der Vinylimidazol-Einheiten und weitere Struktureinheiten aus der Gruppe der Acrylamid- und/oder Methacrylamid-Einheiten.

Weitere besonders bevorzugte erfindungsgemäße Mittel sind dadurch gekennzeichnet, daß sie als kationisches filmbildendes und/oder kationisches festigendes Polymer (b) mindestens ein Copolymer (b3) enthalten, das mindestens eine Struktureinheit gemäß Formel (M1-a) und mindestens eine Struktureinheit gemäß Formel (I) und mindestens eine Struktureinheit gemäß Formel (VII) und mindestens eine Struktureinheit gemäß Formel (VIII) enthält

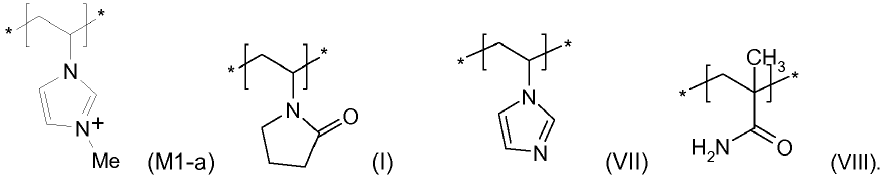

Auch hierbei ist besonders bevorzugt, wenn die Copolymere (b3) neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b3) ausschließlich aus Struktureinheiten der Formel (M1-a), (I), (VII) und (VIII) aufgebaut und lassen sich durch die allgemeine Formel (Poly3) 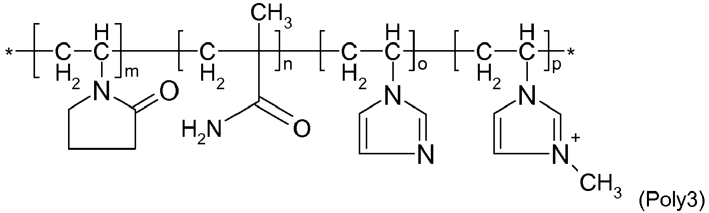 beschreiben, wobei die Indices m, n, o und p je nach Molmasse des Polymers variieren und nicht bedeuten sollen, daß es sich um Blockcopolymere handelt. Vielmehr können Struktureinheiten der Formeln (M1-a), (I), (VII) und (VIII) im Molekül statistisch verteilt vorliegen.

Zur Kompensation der positiven Polymerladung der Komponente (b2) dienen alle möglichen physiologisch verträglichen Anionen, wie beispielsweise Chlorid, Bromid, Hydrogensulfat, Methylsulfat, Ethylsulfat, Tetrafluoroborat, Phosphat, Hydrogenphosphat, Dihydrogenphosphat oder p-Toluolsulfonat, Triflat.

Wird in zur Kompensation der positiven Ladung des Polymer der Formel (Poly3) ein Methosulfat verwendet werden solche N-Methylvinylimidazol/Vinylpyrrolidon/Vinylimidazol/Methacrylamid-Copolymere laut INCI-Nomenklatur als Polyquaternium-68 bezeichnet und sind beispielsweise von der BASF unter dem Handelsnamen Luviquat^{®} Supreme erhältlich.

Ganz besonders bevorzugte Copolymere (b3) enthalten 1 bis 12 Mol-%, vorzugsweise 3 bis 9 Mol.-% und insbesondere 6 Mol.-% Struktureinheiten gemäß Formel (M1-a) und 45 bis 65 Mol.-%, vorzugsweise 50 bis 60 Mol.-% und insbesondere 55 Mol.-% Struktureinheiten gemäß Formel (I) und 1 bis 20 Mol.-%, vorzugsweise 5 bis 15 Mol.-% und insbesondere 10 Mol.-% Struktureinheiten gemäß Formel (VII) und 20 bis 40 Mol.-%, vorzugsweise 25 bis 35 Mol.-% und insbesondere 29 Mol.-% Struktureinheiten gemäß Formel (VIII).

Besonders bevorzugte erfindungsgemäße Mittel enthalten ein Copolymer (b3), das Molmassen innerhalb eines bestimmten Bereiches aufweist. Hier sind erfindungsgemäße Mittel bevorzugt, bei denen das Copolymer (b3) eine Molmasse von 100 bis 500 kDa, vorzugsweise von 150 bis 400 kDa, weiter bevorzugt von 250 bis 350 kDa und insbesondere von 290 bis 310 kDa aufweist.

Unter den zusätzlichen filmbildenden kationischen und/oder festigenden Polymer ausgewählt aus den kationischen Polymeren (b) mit mindesten einem Strukturelement der obigen Formel (M1), gelten als bevorzugt:
- Vinylpyrrolidon/1-Vinyl-3-methyl-1H-imidazoliumchlorid-Copolymere (wie beispielsweise das mit der INCI-Bezeichnung Polyquaternium-16 unter den Handelsbezeichnungen Luviquat^{®} Style, Luviquat® FC 370, Luviquat® FC 550, Luviquat® FC 905 und Luviquat® HM 552 (BASF SE)),
- Vinylpyrrolidon/1-Vinyl-3-methyl-1H-imidazoliummethylsulfat-Copolymere (wie beispielsweise das mit der INCI-Bezeichnung Polyquaternium-44 unter den Handelsbezeichnungen Luviquat^{®} Care (BASF SE)),
- Vinylpyrrolidon/Vinylcaprolactam/1-Vinyl-3-methyl-1H-imidazolium-Terpolymer (wie beispielsweise das mit der INCI-Bezeichnung Polyquaternium-46 unter den Handelsbezeichnungen Luviquat^{®} Care oder Luviquat^{®} Hold (BASF SE)),
- Vinylpyrrolidon/Methacrylamid/Vinylimidazol/1-Vinyl-3-methyl-1H-imidazoliummethylsulfat-Copolymer (wie beispielsweise das mit der INCI-Bezeichnung Polyquaternium-68 unter der Handelsbezeichnung Luviquat^{®} Supreme (BASF SE)),
   sowie Gemische aus diesen Polymeren.

Im Rahmen dieser Ausführungsformen eignen sich die zuvor genannten bevorzugten Ausführungsformen des amphiphilen, kationischen Polymers (a) als bevorzugt (*vide supra*). Gleichsam gelten alle zuvor genannten bevorzugten Mengenangaben bezüglich der Polymerkomponenten (a) und (b) des erfindungsgemäßen Mittels auch für diese Ausführungsformen *mutatis mutandis* als bevorzugt.

Zusätzlich enthält das erfindungsgemäße Mittel zwingend mindestens ein filmbildendes nichtionisches und/oder festigendes nichtionisches Polymer (c). Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Die filmbildenden nichtionischen und/oder festigenden nichtionischen Polymere (c) sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten.

Die filmbildenden nichtionischen und/oder festigenden nichtionischen Polymere (c) werden wiederum bevorzugt ausgewählt aus mindestens einem Polymer der Gruppe, die gebildet wird, aus Homopolymeren und nichtionischen Copolymeren des N-Vinylpyrrolidons.

Geeignete Polyvinylpyrrolidone sind beispielsweise Handelsprodukte wie Luviskol^{®} K 90 oder Luviskol^{®} K 85 der Firma BASF SE.

Mittel, die als filmbildendes nichtionisches und/oder festigendes nichtionisches Polymer (c) mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
sind erfindungsgemäß ganz besonders bevorzugt.

Es gelten weiterhin insbesondere solche Mittel im Rahmen dieser Ausführungsform als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 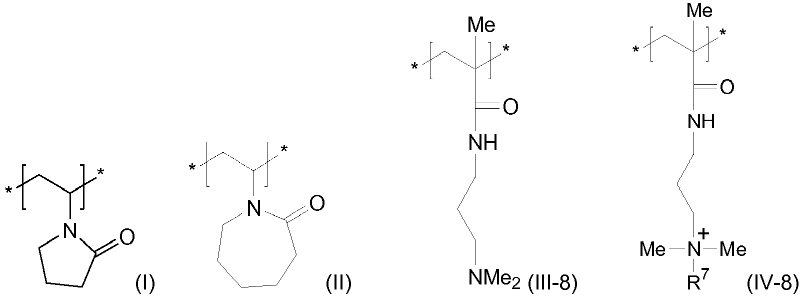 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein weiteres filmbildendes kationisches und/oder festigendes kationisches Polymer
   und
(c) Polyvinylpyrrolidon.

Es gelten weiterhin insbesondere solche Mittel im Rahmen dieser Ausführungsform als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 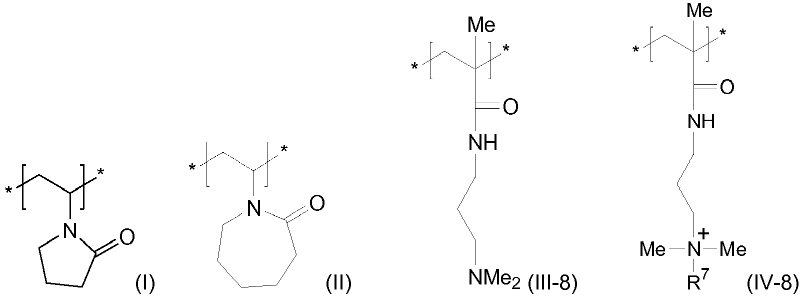 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein weiteres filmbildendes kationisches und/oder festigendes kationisches Polymer
   und
(c) ein Copolymer, welches aus den Monomeren N-Vinylpyrrolidon und Vinylacetat - insbesondere aus keinem weiteren Monomeren - hergestellt wird.

Dabei ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt.

Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Weitere bevorzugte erfindungsgemäße Mittel sind dadurch gekennzeichnet, daß sie als nichtionisches filmbildendes und/oder nichtionisches festigendes Polymer (c) mindestens ein Copolymer (c1) enthalten, das mindestens weitere Struktureinheit gemäß Formel (I) und mindestens eine Struktureinheit gemäß Formel (VII) und mindestens eine Struktureinheit gemäß Formel (VIII) enthält

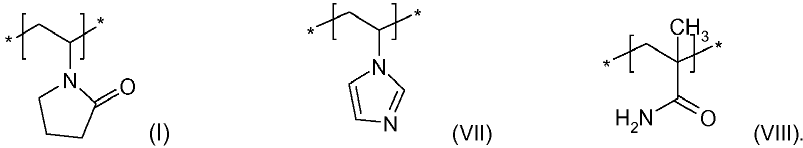

Auch hierbei ist besonders bevorzugt, wenn diese Copolymere neben Polymereinheiten, die aus dem Einbau der genannten Struktureinheiten gemäß Formel (M1-a), (I), (VII) und (VIII) in das Copolymer resultieren, maximal 5 Gew.-%, vorzugsweise maximal 1 Gew.-%, Polymereinheiten enthalten, die auf den Einbau anderer Monomere zurückgehen. Vorzugsweise sind die Copolymere (b3) ausschließlich aus Struktureinheiten der Formel (M1-a), (I), (VII) und (VIII) aufgebaut und lassen sich durch die allgemeine Formel (Poly4) 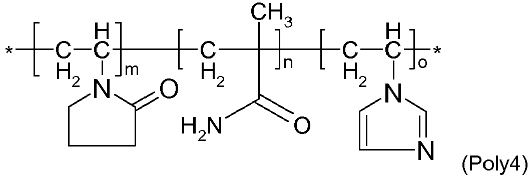 beschreiben, wobei die Indices m, n, o und p je nach Molmasse des Polymers variieren und nicht bedeuten sollen, daß es sich um Blockcopolymere handelt. Vielmehr können Struktureinheiten der Formeln (I), (VII) und (VIII) im Molekül statistisch verteilt vorliegen.

Es gelten weiterhin insbesondere solche Mittel im Rahmen dieser Ausführungsform als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 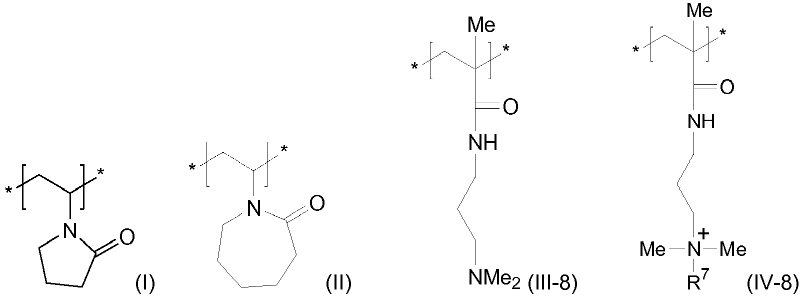 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein weiteres filmbildendes kationisches und/oder festigendes kationisches Polymer und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

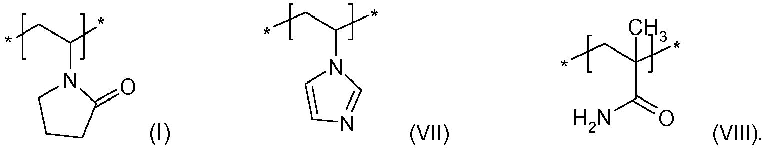

Ein besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Weiterhin hat es sich als bevorzugt gezeigt, zur Lösung der Aufgabe Mittel zu verwenden, die mindestens ein nichtionisches filmbildendes und/oder nichtionisches festigendes Polymer (c) enthalten, umfassend mindestens eine Struktureinheit der Formel (I) und mindestens eine Struktureinheit der Formel (III) 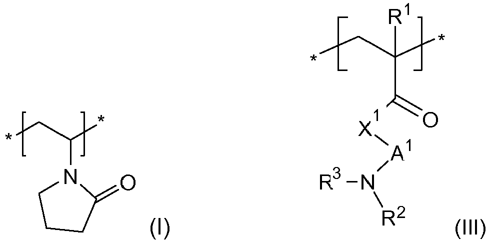 worin
R¹ für ein Wasserstoffatom oder eine Methylgruppe steht,
X¹ für ein Sauerstoffatom oder eine Gruppe NH steht,
A¹ für eine Gruppe Ethan-1,2-diyl, Propan-1,3-diyl oder Butan-1,4-diyl steht
R² und R³ stehen unabhängig voneinander für eine (C₁ bis C₄)-Alkylgruppe.

Es gelten weiterhin insbesondere solche Mittel im Rahmen dieser Ausführungsform als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 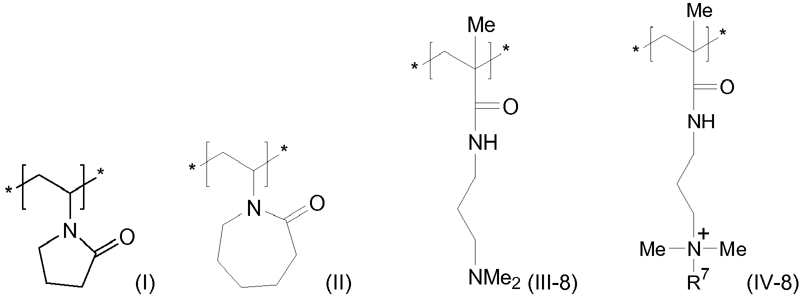 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein weiteres filmbildendes kationisches und/oder festigendes kationisches Polymer
   und
(c) mindestens ein nichtionisches filmbildendes und/oder nichtionisches festigendes Polymer, umfassend mindestens eine Struktureinheit der Formel (I) und mindestens eine Struktureinheit der Formel (III) 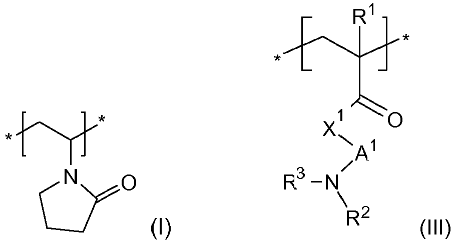 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Dabei ist es insbesondere bevorzugt, wenn das nichtionische filmbildende und/oder nichtionische festigende Polymer ausgewählt wird aus mindestens einem Polymer, welches mindestens eines oder mehrere der folgenden Merkmale erfüllt:
- R¹ bedeutet eine Methylgruppe,
- X¹ steht für eine Gruppe NH,
- A¹ steht für Ethan-1,2-diyl oder Propan-1,3-diyl,
- R² und R³, stehen unabhängig voneinander für Methyl oder Ethyl, (besonders bevorzugt für Methyl).

Besonders bevorzugt handelt es sich bei dem nichtionischen filmbildenden und/oder nichtionischen festigenden Polymer dieser Ausführungsform um mindestens ein Polymer, das mindestens eine Struktureinheit der Formel (I) und mindestens eine Struktureinheit der Formel (III-8) umfasst,

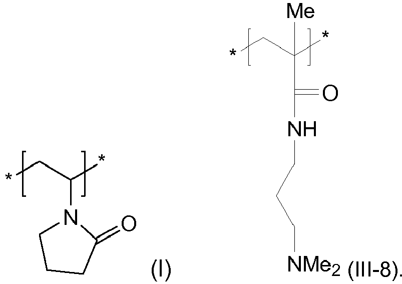

Ein ganz besonders bevorzugtes nichtionisches filmbildendes und/oder nichtionisches festigendes Polymer dieser Ausführungsform ist ein Copolymer aus N-Vinylpyrrolidon und N,N-Dimethylaminiopropylmethacrylamid, welches beispielsweise mit der INCI-Bezeichung VP/DMAPA Acrylates Copolymer z.B. unter der Handelsbezeichnung Styleze^{®} CC 10 von der Firma ISP verkauft wird.

Im Rahmen all dieser Ausführungsformen eignen sich die zuvor genannten bevorzugten Ausführungsformen des amphiphilen, kationischen Polymers (a) als bevorzugt (*vide supra*). Gleichsam gelten alle zuvor genannten bevorzugten Mengenangaben bezüglich der Polymerkomponenten (a) und (b) und (c) des erfindungsgemäßen Mittels auch für diese Ausführungsformen *mutatis mutandis* als bevorzugt.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 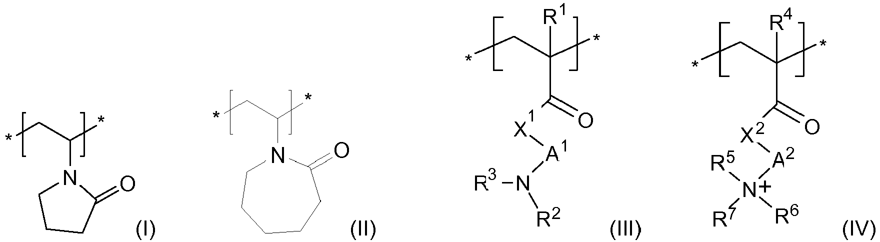 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer ausgewählt aus der Gruppe der kationischen, quaternisierten Cellulosederivate,
   und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 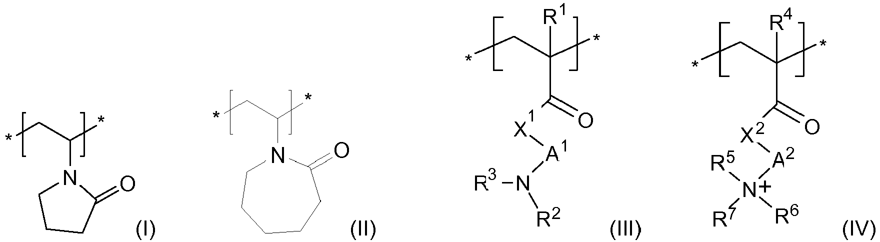 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer ausgewählt aus der Gruppe der kationischen, quaternisierten Cellulosederivate,
   und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 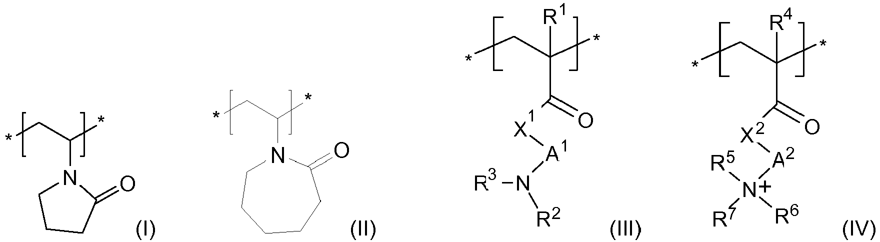 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer ausgewählt aus der Gruppe der kationischen, quaternisierten Cellulosederivate,
   und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

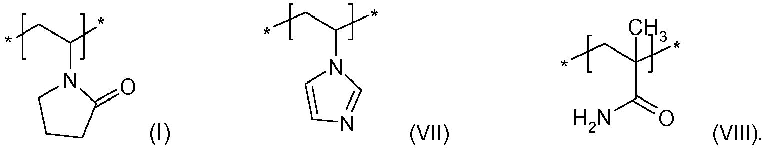

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 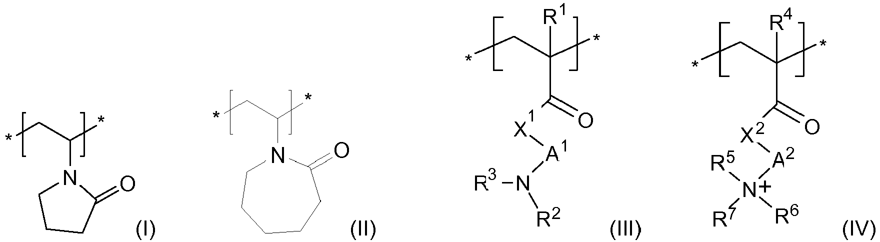 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer ausgewählt aus der Gruppe der kationischen, quaternisierten Cellulosederivate,
   und
(c) mindestens ein nichtionisches filmbildendes und/oder nichtionisches festigendes Polymer, umfassend mindestens eine Struktureinheit der Formel (I) und mindestens eine Struktureinheit der Formel (III) 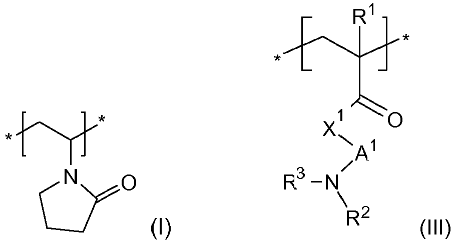 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 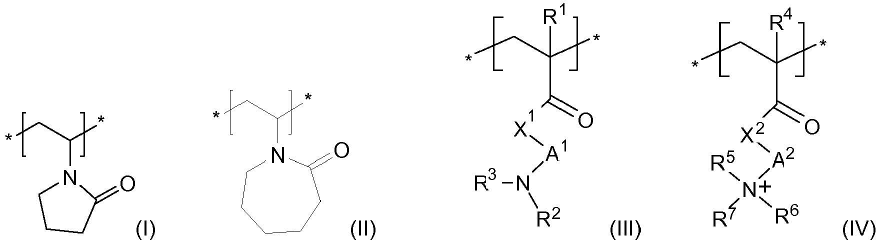 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 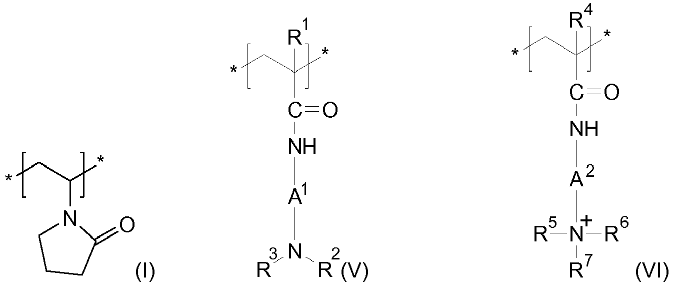 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 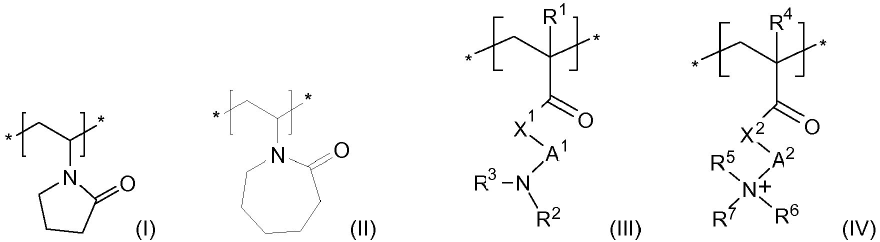 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 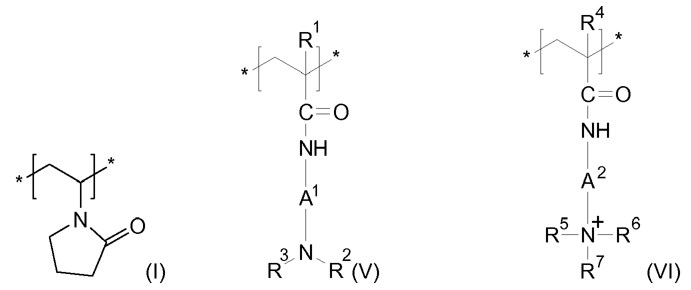 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 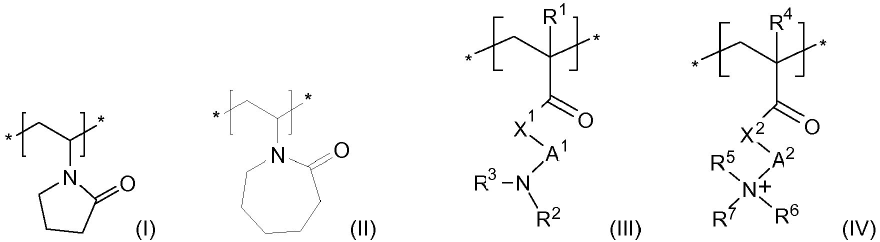 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 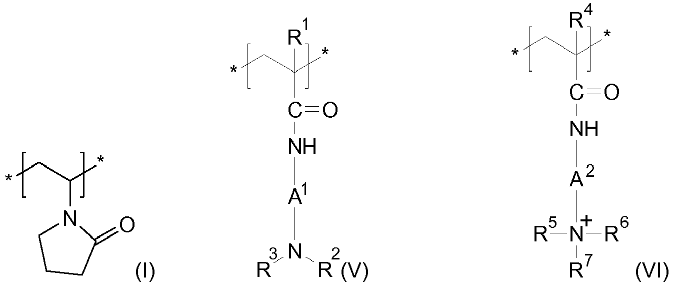 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

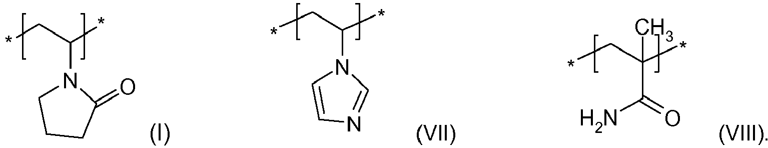

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 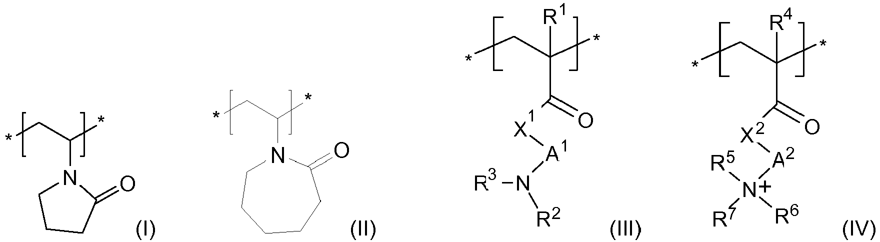 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 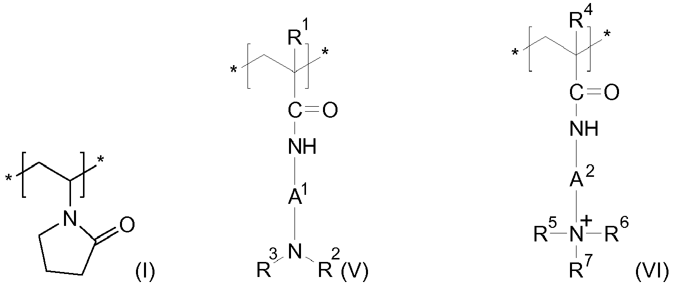 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) mindestens ein nichtionisches filmbildendes und/oder nichtionisches festigendes Polymer, umfassend mindestens eine Struktureinheit der Formel (I) und mindestens eine Struktureinheit der Formel (III) 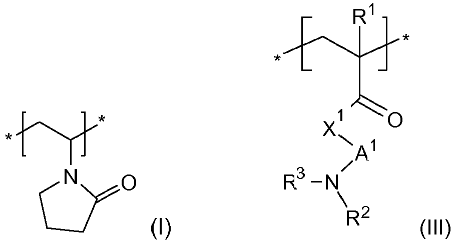 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 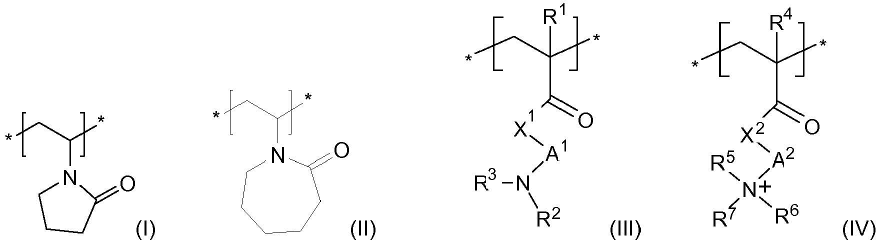 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) enthält 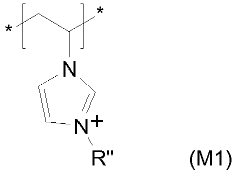 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 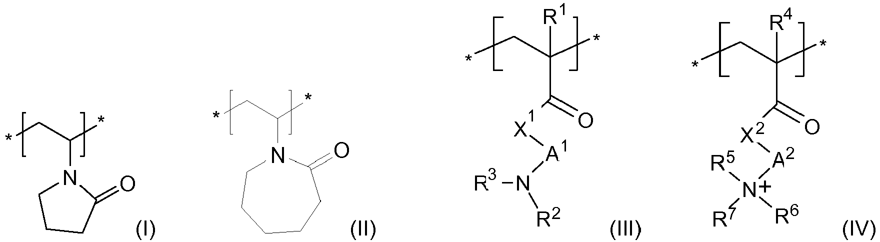 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) enthält 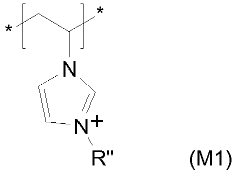 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
   (c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 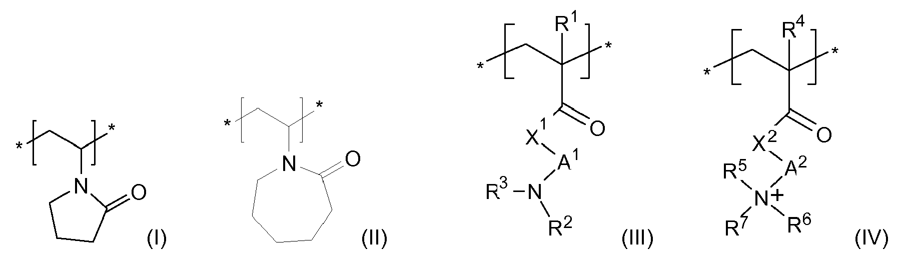 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) enthält 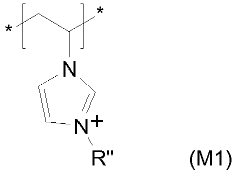 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

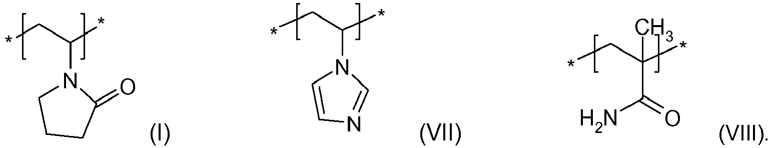

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 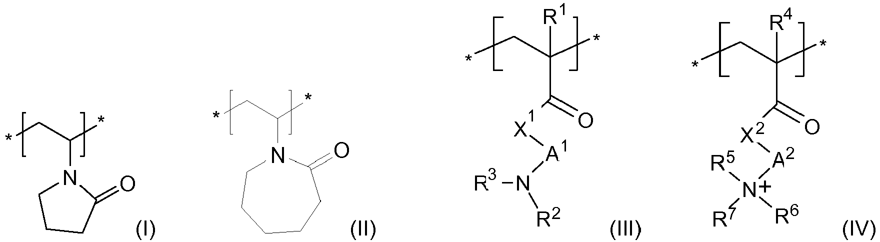 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) enthält 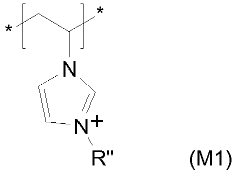 worin
   R für eine (C₁ bis C₄)-Alkylgruppe, insbesondere eine Methylgruppe, steht, und zusätzlich mindestens ein weiteres kationisches und/oder nichtionisches Strukturelement aufweist,
      und
(c) mindestens ein nichtionisches filmbildendes und/oder nichtionisches festigendes Polymer, umfassend mindestens eine Struktureinheit der Formel (I) und mindestens eine Struktureinheit der Formel (III) 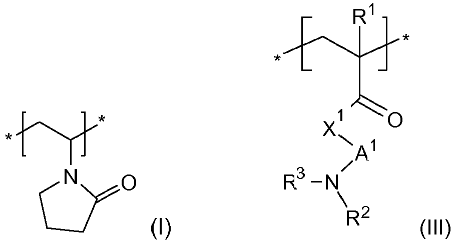 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 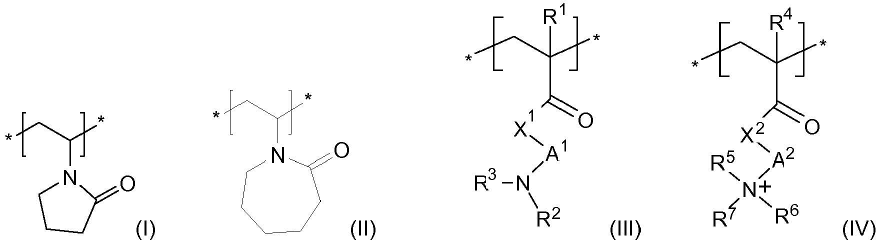 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) und mindestens ein Strukturelement der Formel (I) umfasst 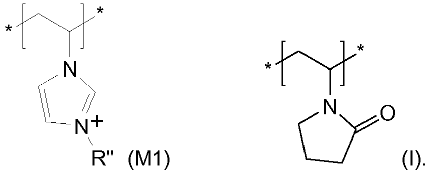 worin
   R für eine (C₁ bis C₄)-Alkylgruppe, insbesondere eine Methylgruppe, steht und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 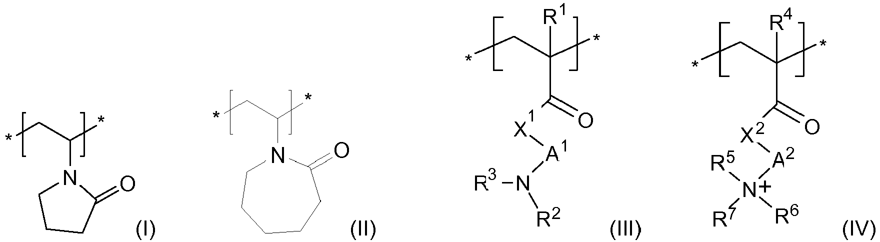 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) und mindestens ein Strukturelement der Formel (I) umfasst 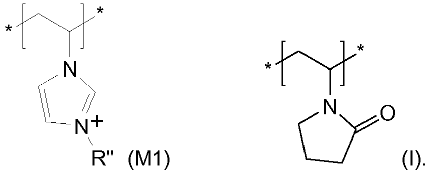 worin
   R für eine (C₁ bis C₄)-Alkylgruppe, insbesondere eine Methylgruppe, steht
      und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 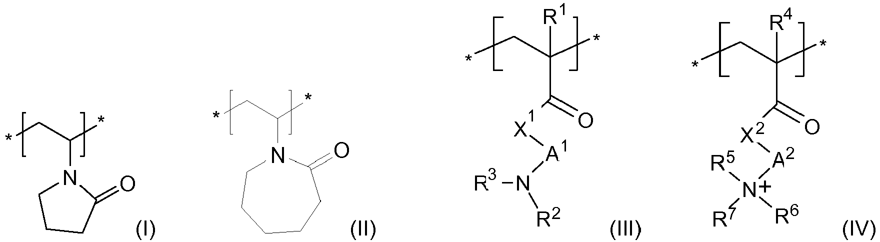 worin
   R¹ und R⁴ stehen unabhängig voneinander für ein Wasserstoffatom oder eine Methylgruppe, X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) und mindestens ein Strukturelement der Formel (I) umfasst 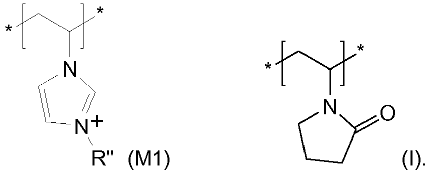 worin
   R für eine (C₁ bis C₄)-Alkylgruppe, insbesondere eine Methylgruppe, steht
      und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

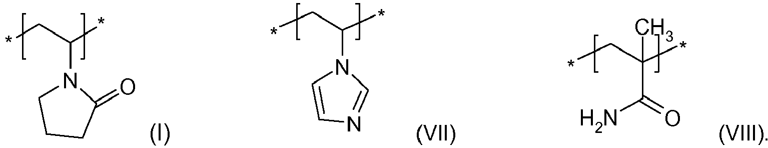

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 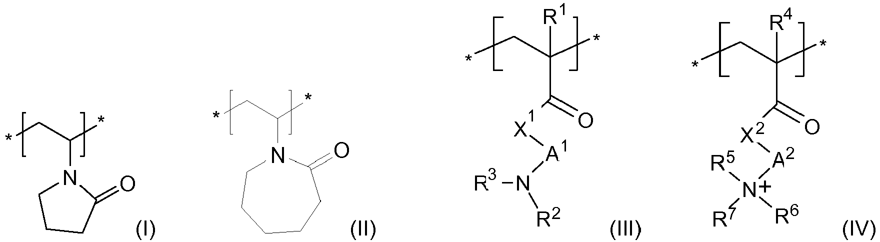 worin
   R¹ und R⁴ stehen unabhängig voneinander für ein Wasserstoffatom oder eine Methylgruppe,
   X¹ und X² stehen unabhängig voneinander für ein Sauerstoffatom oder eine Gruppe NH,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe
      und
(b) mindestens ein kationisches filmbildendes und/oder kationisches festigendes Polymer, das mindestens ein Strukturelement der Formel (M1) und mindestens ein Strukturelement der Formel (I) umfasst 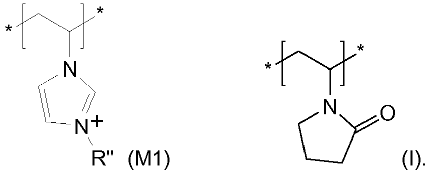 worin
   R für eine (C₁ bis C₄)-Alkylgruppe, insbesondere eine Methylgruppe, steht
      und
(c) mindestens ein nichtionisches filmbildendes und/oder nichtionisches festigendes Polymer, umfassend mindestens eine Struktureinheit der Formel (I) und mindestens eine Struktureinheit der Formel (III) 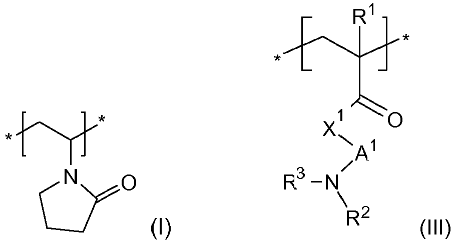 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 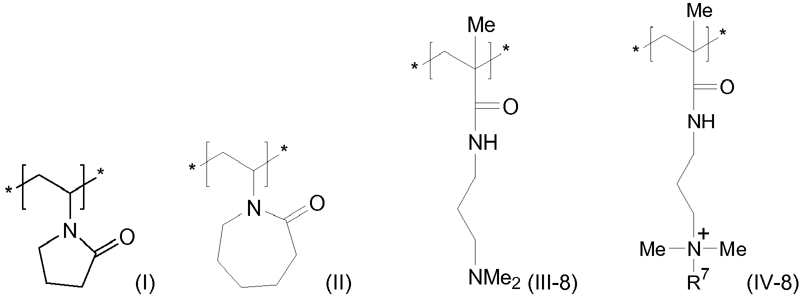 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer ausgewählt aus der Gruppe der kationischen, quaternisierten Cellulosederivate,
   und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 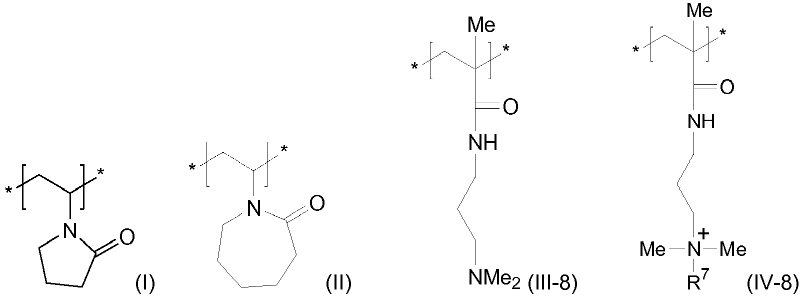 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer ausgewählt aus der Gruppe der kationischen, quaternisierten Cellulosederivate,
   und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 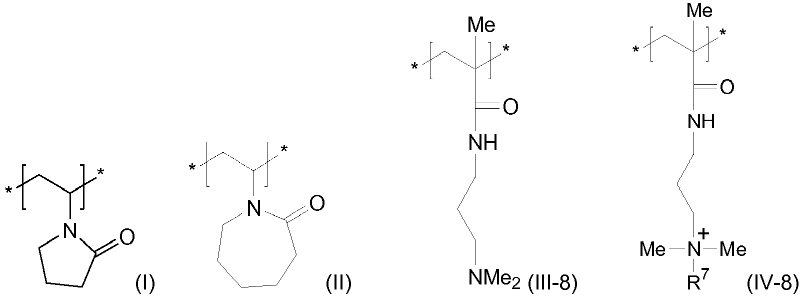 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer ausgewählt aus der Gruppe der kationischen, quaternisierten Cellulosederivate,
   und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

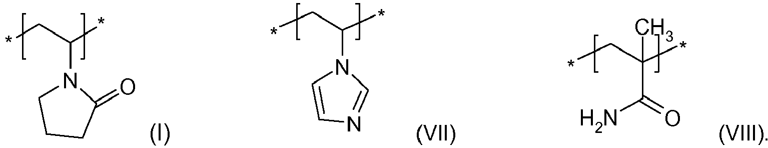

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 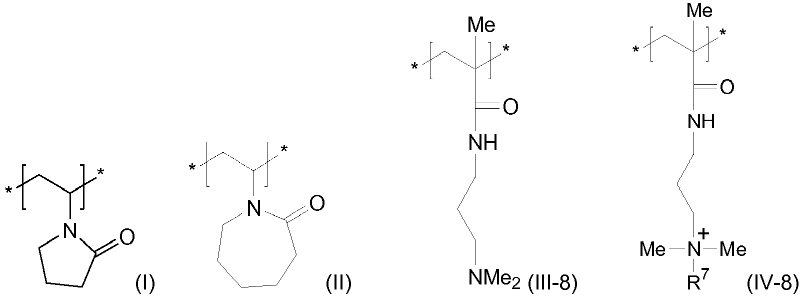 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer ausgewählt aus der Gruppe der kationischen, quaternisierten Cellulosederivate,

   und
(c) mindestens ein nichtionisches filmbildendes und/oder nichtionisches festigendes Polymer, umfassend mindestens eine Struktureinheit der Formel (I) und mindestens eine Struktureinheit der Formel (III) 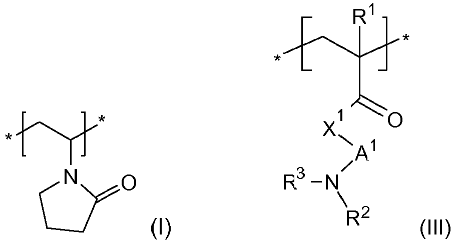 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 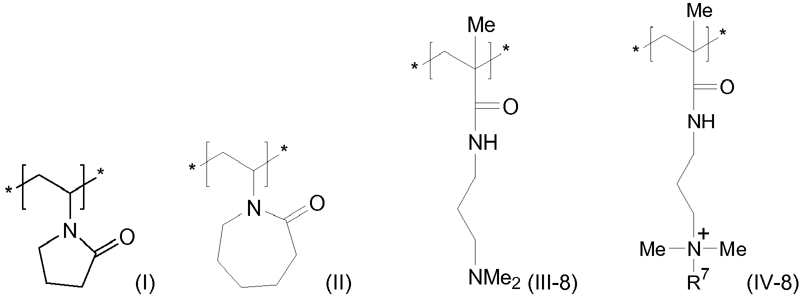 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 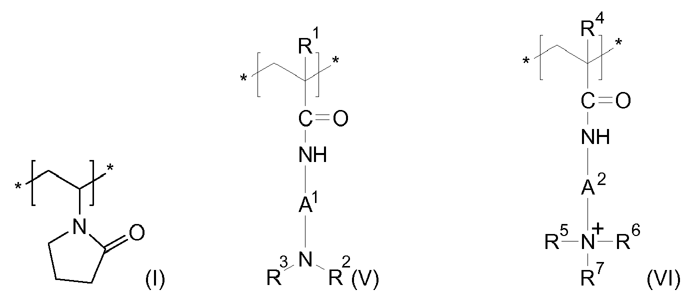 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 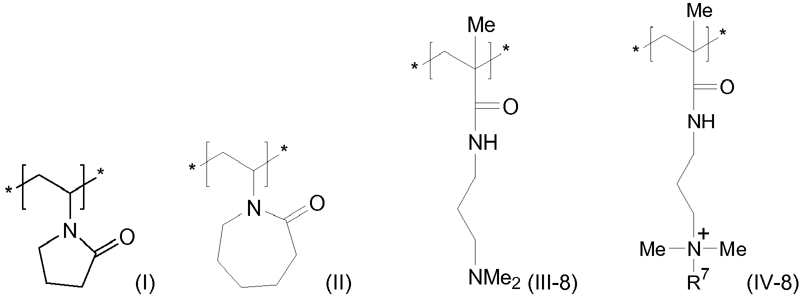 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 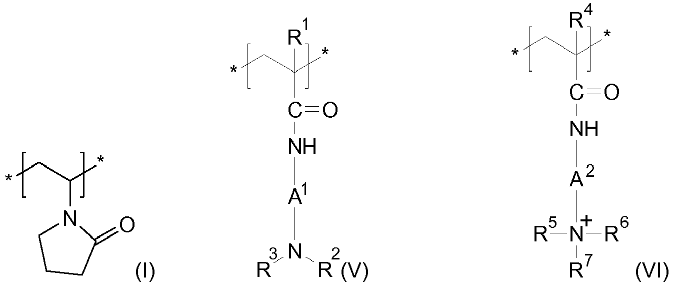 worin
   R¹ und R⁴ stehen unabhängig voneinander für ein Wasserstoffatom oder eine Methylgruppe, A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 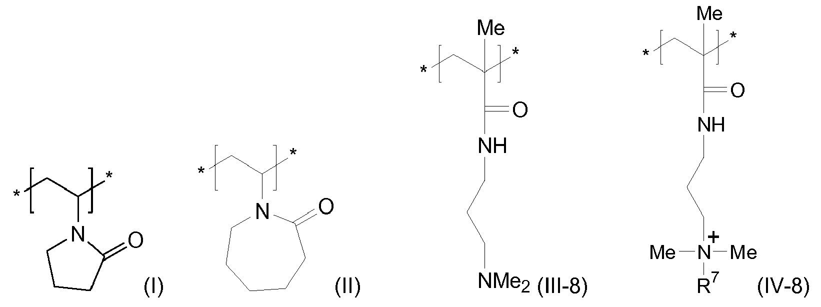 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 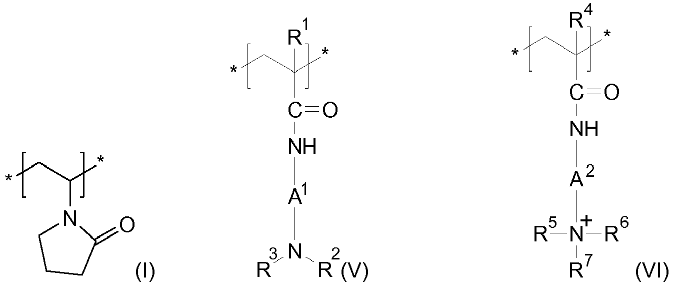 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

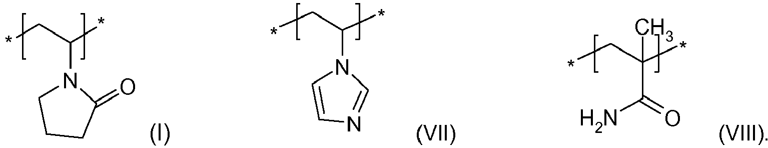

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 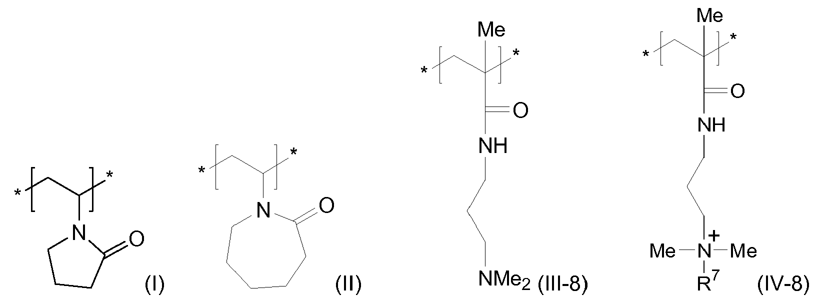 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer, umfassend mindestens eine Struktureinheit der Formel (I) und mindestens eine Struktureinheit der Formel (VI) und gegebenenfalls mindestens eine Struktureinheit der Formel (V) 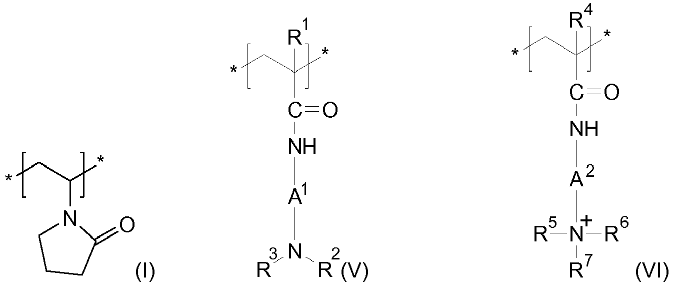 worin
   R¹ und R⁴ stehen unabhängig voneinander für ein Wasserstoffatom oder eine Methylgruppe,
   A¹ und A² stehen unabhängig voneinander für eine Gruppe Ethan-1,2-diyl, Propan-1,3-diyl oder Butan-1,4-diyl,
   R², R³, R⁵ und R⁶ stehen unabhängig voneinander für eine (C₁ bis C₄)-Alkylgruppe,
   R⁷ steht für eine (C₈ bis C₃₀)-Alkylgruppe.
      und
(c) mindestens ein nichtionisches filmbildendes und/oder nichtionisches festigendes Polymer, umfassend mindestens eine Struktureinheit der Formel (I) und mindestens eine Struktureinheit der Formel (III) 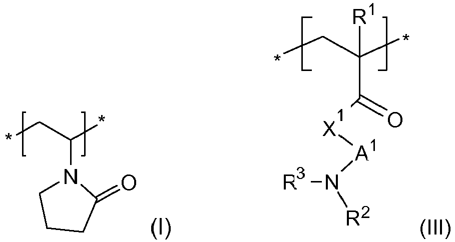 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 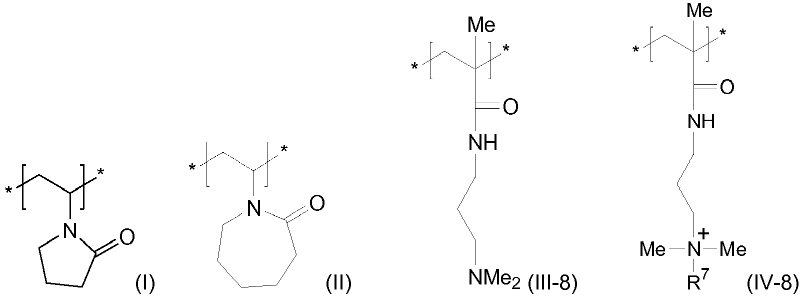 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein zusätzliches filmbildendes kationisches und/oder festigendes kationisches Polymer, umfassend mindestens eine Struktureinheit der Formel (I) und mindestens eine Struktureinheit der Formel (VI-8) und gegebenenfalls mindestens eine Struktureinheit der Formel (III-8) 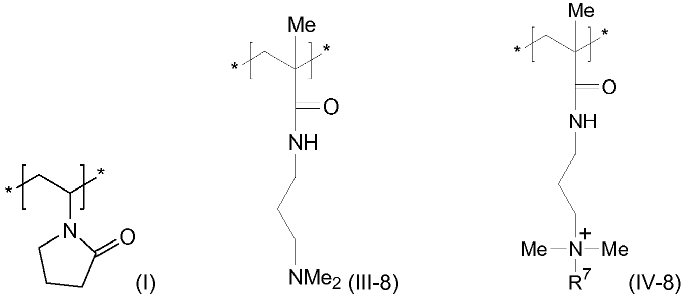 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(c) mindestens ein nichtionisches filmbildendes und/oder nichtionisches festigendes Polymer, umfassend mindestens eine Struktureinheit der Formel (I) und mindestens eine Struktureinheit der Formel (III-8) 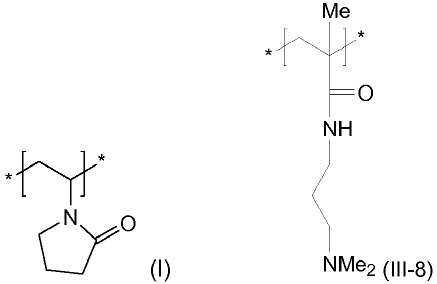 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Ganz besonders bevorzugt sind folgende Polymerkombinationen:
(a) Copolymer aus N-Vinylpyrrolidon/N-Vinylcaprolactam/N-(3-Dimethylaminopropyl)methacrylamid und 3-(Methacryloylamino)propyl-lauryl-dimethylammoniumchlorid,
(b) Copolymer aus N-Vinylpyrrolidon, N,N-Dimethylaminopropylmethacrylamid und N,N-Dimethyl-N-dodecylammoniopropylmethacrylamid chlorid,
(c) Copolymer aus N-Vinylpyrrolidon und N,N-Dimethylaminopropylmethacrylamid.

(a) Copolymer aus N-Vinylpyrrolidon/N-Vinylcaprolactam/N-(3-Dimethylaminopropyl)methacrylamid und 3-(Methacryloylamino)propyl-lauryl-dimethylammoniumchlorid,
(b) Dimethylaminoethylmethacrylat-Vinylpyrrolidon-Copolymer, quaterniert mit Diethylsulfat,
(c) Copolymer aus N-Vinylpyrrolidon und N,N-Dimethylaminopropylmethacrylamid.

Obige Polymerkombinationen haben die INCI-Bezeichnungen:
(a) Polyquaternium-69 (b) Polyquaternium-55 (c) VP/DMAPA Acrylates Copolymer
(a) Polyquaternium-69 (b) Polyquaternium-11 (c) VP/DMAPA Acrylates Copolymer

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 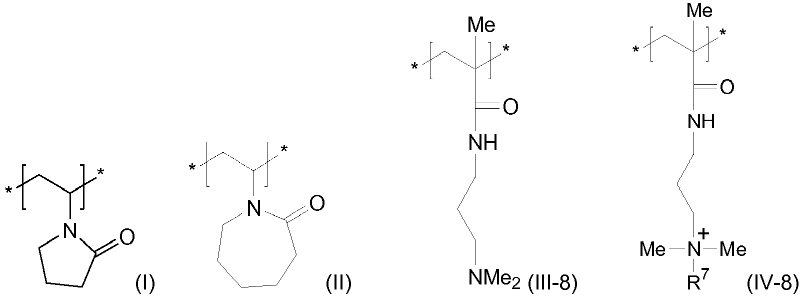 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) enthält 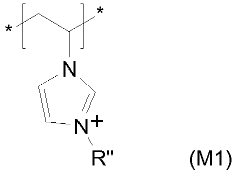 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 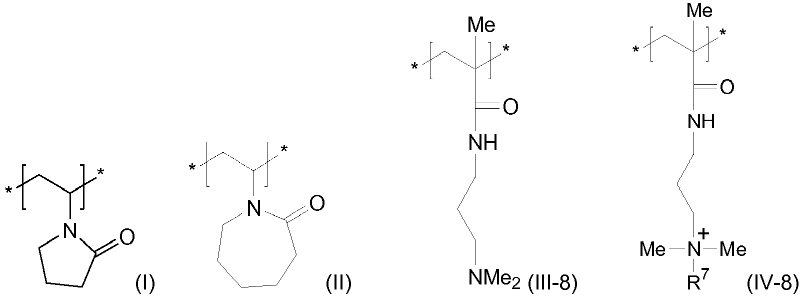 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) enthält 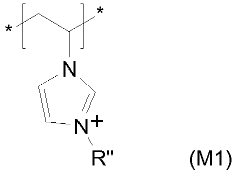 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 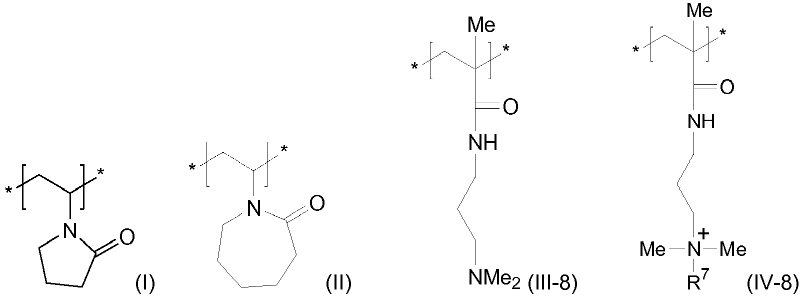 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) enthält 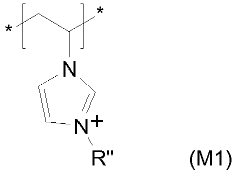 worin
   R für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

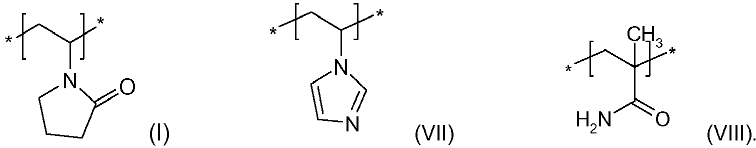

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 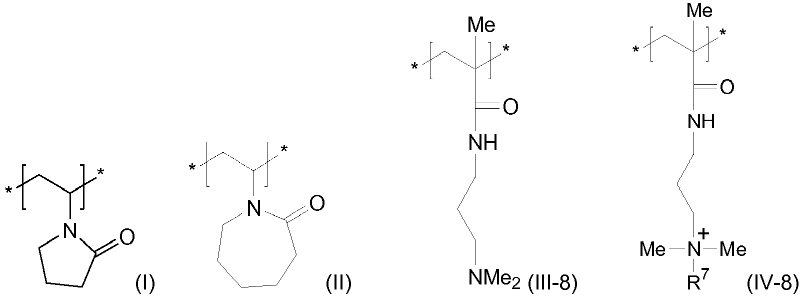 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) enthält 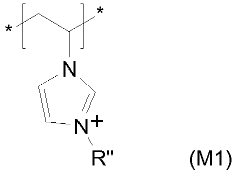 worin
   R^{"} für eine (C₁ bis C₄)-Alkylgruppe, insbesondere eine Methylgruppe, steht,
      und zusätzlich mindestens ein weiteres kationisches und/oder nichtionisches Strukturelement aufweist,
      und
(c) mindestens ein nichtionisches filmbildendes und/oder nichtionisches festigendes Polymer, umfassend mindestens eine Struktureinheit der Formel (I) und mindestens eine Struktureinheit der Formel (III) 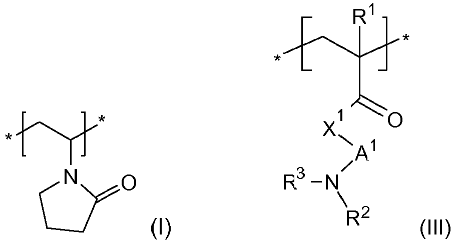 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 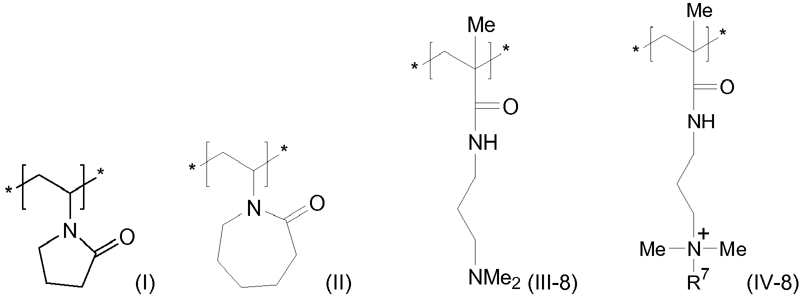 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) und mindestens ein Strukturelement der Formel (I) umfasst 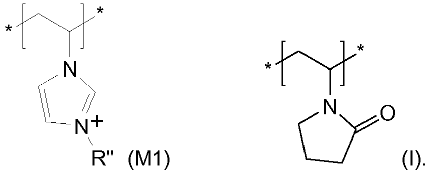 worin
   R für eine (C₁ bis C₄)-Alkylgruppe, insbesondere eine Methylgruppe, steht
   und
(c) Polyvinylpyrrolidon.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 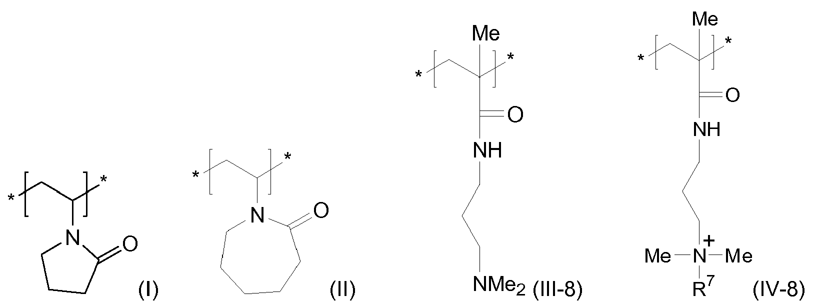 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) und mindestens ein Strukturelement der Formel (I) umfasst 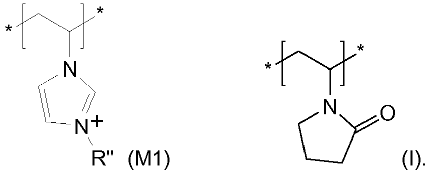 worin
   R für eine (C₁ bis C₄)-Alkylgruppe, insbesondere eine Methylgruppe, steht
   und
(c) ein Copolymer, welches aus den Monomeren N-Vinylpyrrolidon und Vinylacetat - insbesondere aus keinem weiteren Monomeren - hergestellt wird.

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 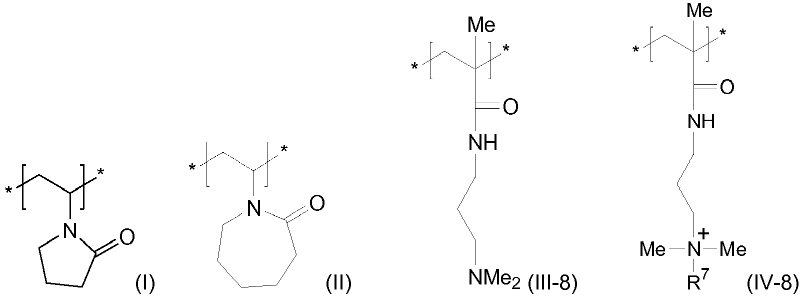 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) und mindestens ein Strukturelement der Formel (I) umfasst 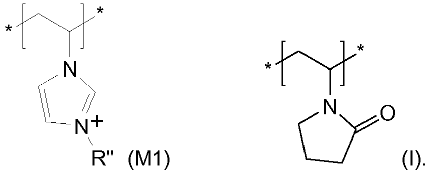 worin
   R für eine (C₁ bis C₄)-Alkylgruppe, insbesondere eine Methylgruppe, steht

   und
(c) mindestens ein nichtionisches filmbildendes und/oder nichtionisches festigendes Polymer, umfassend Struktureinheiten gemäß Formel (I) und Struktureinheiten gemäß Formel (VII) und Struktureinheiten gemäß Formel (VIII)

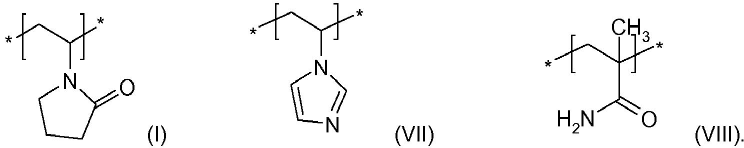

Es gelten weiterhin insbesondere solche Mittel als ganz besonders bevorzugt, die in einem kosmetisch akzeptablen Träger enthalten
(a) mindestens ein amphiphiles, kationisches Polymer, umfassend mindestens eine Struktureinheit der Formel (I), mindestens eine Struktureinheit der Formel (II), mindestens eine Struktureinheit der Formel (III-8) und mindestens eine Struktureinheit der Formel (IV-8), 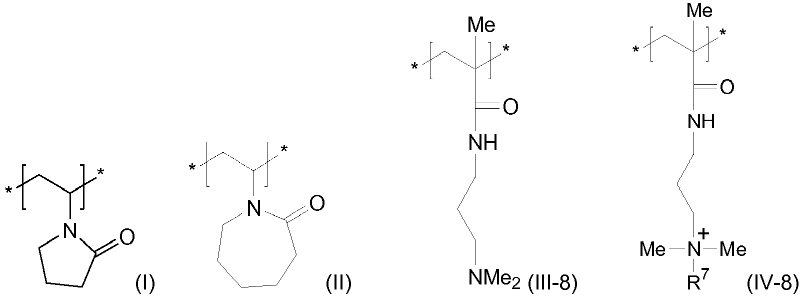 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl),
   und
(b) mindestens ein kationisches filmbildendes und/oder kationisches festigendes Polymer, das mindestens ein Strukturelement der Formel (M1) und mindestens ein Strukturelement der Formel (I) umfasst 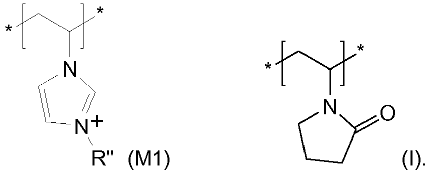 worin
   R für eine (C₁ bis C₄)-Alkylgruppe, insbesondere eine Methylgruppe, steht
   und
(c) mindestens ein nichtionisches filmbildendes und/oder nichtionisches festigendes Polymer, umfassend mindestens eine Struktureinheit der Formel (I) und mindestens eine Struktureinheit der Formel (III) 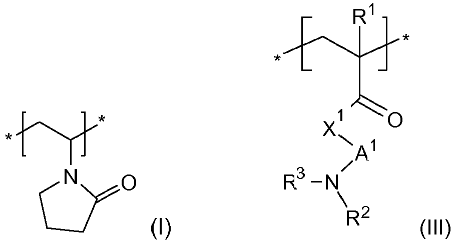 worin
   R¹ für ein Wasserstoffatom oder eine Methylgruppe steht,
   X¹ für ein Sauerstoffatom oder eine Gruppe NH steht,
   A¹ für eine Gruppe Ethan-1,2-diyl, Propan-1,3-diyl oder Butan-1,4-diyl steht
   R² und R³ stehen unabhängig voneinander für eine (C₁ bis C₄)-Alkylgruppe.

Ganz besonders bevorzugt sind folgende Polymerkombinationen:
(a) Copolymer aus N-Vinylpyrrolidon/N-Vinylcaprolactam/N-(3-Dimethylaminopropyl)methacrylamid und 3-(Methacryloylamino)propyl-lauryl-dimethylammoniumchlorid,
(b) 3-Methyl-1-vinylimidazoliumchlorid-Vinylpyrrolidon-Copolymerisat,
(c) Copolymer aus N-Vinylpyrrolidon und N,N-Dimethylaminopropylmethacrylamid.

(a) Copolymer aus N-Vinylpyrrolidon/N-Vinylcaprolactam/N-(3-Dimethylaminopropyl)methacrylamid und 3-(Methacryloylamino)propyl-lauryl-dimethylammoniumchlorid,
(b) Vinylpyrrolidon-Methacrylamid-Vinylimidazol-Vinylimidazoliummethosulfat-Copolymerisat,
(c) Copolymer aus N-Vinylpyrrolidon und N,N-Dimethylaminopropylmethacrylamid.

Die o.g. Polymerkombinationen besitzen die INCI-Bezeichnungen:
(a) Polyquaternium-69 (b) Polyquaternium-16 (c) VP/DMAPA Acrylates Copolymer
(a) Polyquaternium-69 (b) Polyquaternium-68 (c) VP/DMAPA Acrylates Copolymer

Im Rahmen all dieser Ausführungsformen eignen sich die zuvor genannten bevorzugten Ausführungsformen des amphiphilen, kationischen Polymers (a) als bevorzugt (*vide supra*). Gleichsam gelten alle zuvor genannten bevorzugten Mengenangaben bezüglich der Polymerkomponenten (a) und (b) und (c) des erfindungsgemäßen Mittels auch für diese Ausführungsformen *mutatis mutandis* als bevorzugt.

Zur Intensivierung des erfindungsgemäßen Effektes enthalten die erfindungsgemäßen Mittel vorzugsweise zusätzlich mindestens ein Tensid, wobei sich prinzipiell nichtionische, anionische, kationische, ampholytische Tenside eignen. Die Gruppe der ampholytischen oder auch amphoteren Tenside umfasst zwitterionische Tenside und Ampholyte. Die Tenside können erfindungsgemäß bereits emulgierende Wirkung haben.

Die zusätzlichen Tenside sind in dem erfindungsgemäß Mittel bevorzugt in einer Menge von 0,01 Gew.-% bis 5 Gew.-%, besonders bevorzugt von 0,05 Gew.-% bis 0,5 Gew.-%, jeweils bezogen auf das Gewicht des Mittels, enthalten.

Es hat sich als besonders bevorzugt erwiesen, wenn die erfindungsgemäßen Mittel zusätzlich mindestens ein nichtionisches Tensid enthalten.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Amidethercarbonsäuren,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate und Ethercarbonsäuren mit 10 bis 18 C-Atomen in der Alkylgruppe und bis zu 12 Glykolethergruppen im Molekül, Sulfobernsteinsäuremono- und - dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer C₈ - C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das C₁₂ - C₁₈ - Acylsarcosin.

Die erfindungsgemäßen Mittel enthalten die Inhalts- bzw- Wirkstoffe in einem kosmetisch akzeptablen Träger. Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.-% bezogen auf das gesamte Mittel.

Insbesondere der Zusatz von Glycerin und/oder Propylenglykol und/oder Polyethylenglykol und/oder Polypropylenglykol erhöht die Flexibilität des bei Anwendung des erfindungsgemäßen Mittels gebildeten Polymerfilms. Wird also ein flexibler Halt gewünscht, enthalten die erfindungsgemäßen Mittel vorzugsweise 0,01 bis 30 Gew.-% Glycerin und/oder Propylenglykol und/oder Polyethylenglykol und/oder Polypropylenglykol bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise herkömmlichen Stylingmitteln zugesetzt werden. Als geeignete Hilfs- und Zusatzstoffe sind insbesondere zusätzliche Pflegestoffe zu nennen. Als Pflegestoff wird erfindungsgemäß bevorzugt mindestens ein Silikonöl und/oder mindestens ein Silikongum eingesetzt.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Zunächst werden hierunter die Dimethiconole verstanden.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401 DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone bilden die zweite Gruppe der Silikone, welche erfindungsgemäß enthalten sein können. Diese können sowohl linear als auch verzweigt als auch cyclisch oder cyclisch und verzweigt sein. Dimethiconcopolyole bilden eine weitere Gruppe von Silikonen, die geeignet sind. Entsprechende Dimethiconcopolyole sind kommerziell erhältlich und werden beispielsweise von der Firma Dow Corning unter der Bezeichnung Dow Corning ^{®} 5330 Fluid vertrieben.

Selbstverständlich umfasst die erfindungsgemäße Lehre auch, dass die Dimethiconole, Dimethicone und/oder Dimethiconcopolymere bereits als Emulsion vorliegen können. Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Besonders geeignete Silikone sind aminofunktionelle Silikone, insbesondere die Silikone, die unter der INCI-Bezeichnung Amodimethicone zusammengefasst sind. Daher ist es erfindungsgemäß bevorzugt, wenn die erfindungsgemäß Mittel zusätzlich mindestens ein aminofunktionelles Silikon enthalten. Darunter sind Silikone zu verstehen, welche mindestens eine, gegebenenfalls substituierte, Aminogruppe aufweisen. Diese Silikone werden nach der INCI-Deklaration als Amodimethicone bezeichnet und sind beispielsweise in Form einer Emulsion als Handelsprodukt Dow Corning^{®} 939 oder als Handelsprodukt Dow Corning^{®} 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Die Mittel enthalten die Silikone bzw. Silikongums bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel optional mindestens ein Proteinhydrolysat und/oder eines seiner Derivate enthalten. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff kann das erfindungsgemäße Mittel weiterhin mindestens ein Vitamin, ein Provitamin, eine Vitaminvorstufe und/oder eines derer Derivate enthalten.

Dabei sind erfindungsgemäß solche Vitamine, Provitamine und Vitaminvorstufen bevorzugt, die üblicherweise den Gruppen A, B, C, E, F und H zugeordnet werden. Zur Gruppe der als Vitamin A bezeichneten Substanzen gehören das Retinol (Vitamin A₁) sowie das 3,4-Didehydroretinol (Vitamin A₂). Zur Vitamin B-Gruppe oder zu dem Vitamin B-Komplex gehören u. a. Vitamin B₁ (Thiamin), Vitamin B₂ (Riboflavin), Vitamin B₃ (Nicotinsäure und/oder Nicotinsäureamid (Niacinamid)), Vitamin B₅ (Pantothensäure, Panthenol und Pantolacton), Vitamin B₆ (Pyridoxin sowie Pyridoxamin und Pyridoxal). Weitere Vertreter der o.g. Vitamine sind Vitamin C (Ascorbinsäure), Vitamin E (Tocopherole, insbesondere α-Tocopherol), Vitamin F (Linolsäure und/oder Linolensäure), Vitamin H.

Bevorzugt enthalten die erfindungsgemäßen Mittel Vitamine, Provitamine und Vitaminvorstufen aus den Gruppen A, B, C, E und H. Panthenol, Pantolacton, Pyridoxin und seine Derivate sowie Nicotinsäureamid und Biotin sind besonders bevorzugt. Ganz besonders bevorzugt wird als Pflegestoff D-Panthenol, gegebenenfalls in Kombination mit mindestens einem der oben genannten Silikonderivate eingesetzt.

Wird also ein besonders flexibler Halt gewünscht, können die erfindungsgemäßen Mittel statt oder zusätzlich zu Glycerin und/oder Propylenglykol Panthenol enthalten. In einer bevorzugten Ausführungsform enthalten die erfindungsgemäßen Mittel Panthenol, vorzugsweise in einer Menge von 0,05 bis 10 Gew.-%, besonders bevorzugt 0,1 - 5 Gew.-%, jeweils bezogen auf das gesamte Mittel.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Das Mittel kann weiterhin mindestens ein Lipid als Pflegestoff enthalten. Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀-Fettalkoholen. Bevorzugt sind die Monoester der Fettsäuren mit Alkoholen mit 2 bis 24 C-Atomen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 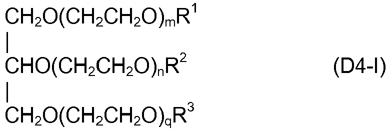
in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Durch Zugabe eines UV-Filters können sowohl die Mittel selbst, als auch die behandelten Fasern vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt. Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt. Direktziehende Farbstoffe sind üblicherweise Nitrophenylendiamine, Nitroaminophenole, Azofarbstoffe, Anthrachinone, Indophenole oder kationische direktziehende Farbstoffe eingesetzt. Besonders bevorzugt sind als kationische Farbstoffe
- kationische Triphenylmethanfarbstoffe, wie beispielsweise Basic Blue 7, Basic Blue 26, Basic Violet 2 und Basic Violet 14,
- aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
- direktziehende Farbstoffe, die einen Heterocyclus enthalten, der mindestens ein quaternäres Stickstoffatom aufweist. Die Farbstoffe, die auch unter den Bezeichnungen Basic Yellow 87, Basic Orange 31 und Basic Red 51 bekannt sind, sind hier ganz besonders bevorzugte kationische direktziehende Farbstoffe.

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe. Weiterhin können die erfindungsgemäßen Mittel auch in der Natur vorkommende Farbstoffe enthalten, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt.

Vorzugsweise werden die erfindungsgemäßen Mittel als Pumpspray, Aerosolspray, Pumpschaum oder Aerosolschaum.

Hierzu werden die erfindungsgemäßen Mittel in einer Abgabevorrichtung konfektioniert, die entweder ein zusätzlich mit einem Treibmittel befüllter Druckgasbehälter ("Aerosolbehälter") oder ein Nichtaerosolbehälter darstellt.

Die Druckgasbehälter, mit deren Hilfe ein Produkt durch den inneren Gasdruck des Behälters über ein Ventil verteilt wird, bezeichnet man definitionsgemäß als "Aerosolbehälter". Als "Nichtaerosolbehälter" wird im Umkehrschluß zur Aerosoldefinition ein Behältnis unter Normaldruck definiert, mit dessen Hilfe ein Produkt mittels mechanischer Einwirkung durch ein Pumpsystem verteilt wird.

Insbesondere bevorzugt sind die erfindungsgemäßen Mittel als Aerosolhaarschaum oder Aerosolhaarspray konfektioniert. Das erfindungsgemäße Mittel enthält daher bevorzugt zusätzlich mindestens ein Treibmittel.

Erfindungsgemäß geeignete Treibmittel sind beispielsweise ausgewählt aus N₂O, Dimethylether, CO₂, Luft, Alkanen mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und Mischungen daraus.

Gemäß einer bevorzugten Ausführungsform werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Zur Verschäumung von gelförmigen Mitteln in einem Zweikammer-Aerosolbehälter eignet sich bevorzugt Isopentan als ein Treibmittel, welches in die erfindungsgemäßen Mittel eingearbeitet wird und in der ersten Kammer des Zweikammer-Aerosolbehälters konfektioniert ist. In der zweiten Kammer des Zweikammer-Aerosolbehälters wird mindestens ein weiteres, von Isopentan verschiedenes Treibmittel konfektioniert, welches in dem Zweikammer-Aerosolbehälter einen höheren Druck aufbaut als das Isopentan. Die Treibmittel der zweiten Kammer werden bevorzugt ausgewählt aus N₂O, Dimethylether, CO₂, Luft, Alkanen mit 3 oder 4 Kohlenstoffatomen (wie Propan, n-Butan, iso-Butan) sowie Mischungen daraus.

Eine bevorzugte Ausführungsform der erfindungsgemäßen Mittel sind Aerosolhaarschäume oder Aerosolhaarsprays, enthaltend das zuvor beschriebene erfindungsgemäße Mittel und zusätzlich mindestens ein Treibmittel.

Bevorzugte erfindungsgemäße Mittel und Treibmittel des Aerosolhaarschaums bzw. Aerosolhaarsprays, sowie die jeweiligen Mengen an Treibmittel entsprechen dem bereits oben Ausgeführten.

Ein zweiter Gegenstand der Erfindung ist die Verwendung der erfindungsgemäßen Mittel zur temporären Verformung von Haaren.

Die erfindungsgemäßen Mittel und Produkte, die diese Mittel enthalten, insbesondere Aerosolhaarschäume bzw. Aerosolhaarsprays, zeichnen sich insbesondere dadurch aus, dass sie behandeltem Haar einen sehr starken, dauerhaften Frisurenhalt verleihen, obgleich das Haar bleibt flexibel bleibt. Wird das Mittel als Haarschaum konfektioniert, bildet sich ein stabiler, feinporiger und cremiger Schaum, der sich gleichmäßig und ohne zu tropfen auf dem Haar verteilen lässt.

Ein dritter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand zu einem Schaum verschäumt wird und der resultierende Schaum auf die keratinhaltigen Fasern appliziert wird, und worin die Fasern in Form gebracht werden und diese Form durch das Mittel des ersten Erfindungsgegenstandes fixiert wird.

Als erfindungsgemäß bevorzugt gelten die zuvor genannten Abgabevorrichtungen (*vide supra*).

Ein vierter Gegenstand der Erfindung ist ein Verfahren zur Behandlung keratinhaltiger Fasern, insbesondere menschlicher Haare, worin unter Einsatz einer Abgabevorrichtung ein Mittel gemäß erstem Erfindungsgegenstand als Spray auf die keratinhaltigen Fasern appliziert wird, und worin die Fasern in Form gebracht werden und diese Form durch das Mittel des ersten Erfindungsgegenstandes fixiert wird.

Als erfindungsgemäß bevorzugt gelten die zuvor genannten Abgabevorrichtungen (*vide supra*).

Die nachfolgenden Beispiele sollen den Gegenstand der vorliegenden Erfindung erläutern ohne ihn in irgendeiner Weise zu beschränken.

### Beispiele

Die folgenden Mengenangaben verstehen sich - soweit nichts anderes vermerkt ist - in Gewichtsprozent.

Es wurden folgende Rezepturen durch Vermischen der angegebenen Rohstoffe bereitgestellt:

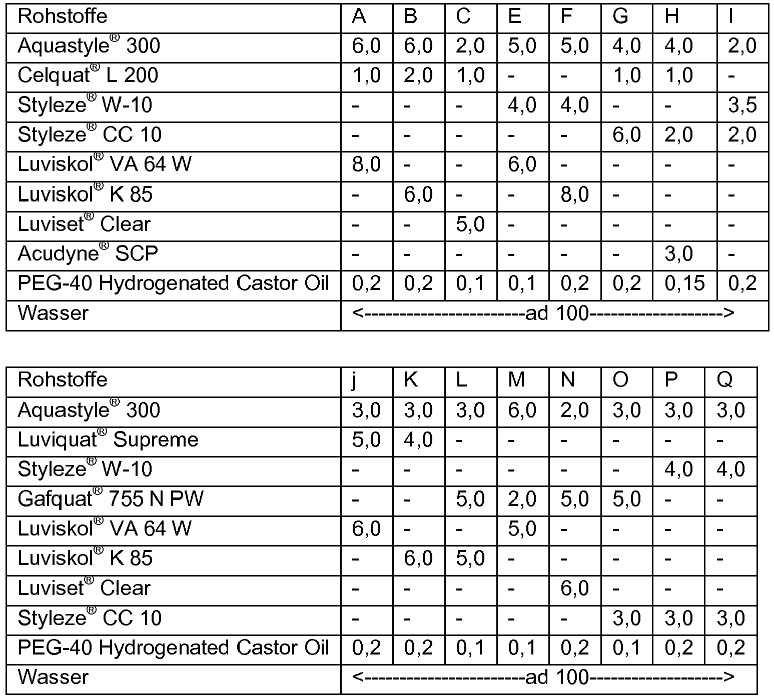

Die Rezepturen A bis Q wurden jeweils in ein Aerosolbehältnis gefüllt, das folgende technische Parameter erfüllt: Aluminium Vorratsbehältnis mit Ventil Produkt 522983 PV10697 der Firma Pecision (Deutsche Präzisions-Ventil GmbH).
Das Aerosolbehältnis wurde mit einem Treibgasgemisch aus Propan/Butan (47 Gew.-% Propan, 50 Gew.-% Butan, 3 Gew.-% Isobutan) befüllt, sodass sich ein Gewichtsverhältnis von Rezeptur zu Treibgas von 92 zu 8 ergab.

Alle Rezepturen bewirkten nach der Anwendung auf dem Haar einen hervorragenden flexiblen Frisurenhalt. Das Haar erhielt eine sehr gute Pflege. Als Aerosolschaum ausgebracht, wurde ein voluminöser Schaum erhalten, der erst während der Anwendung am Haar signifikant bricht.

Verzeichnis der eingesetzten Rohstoffe:
- Aquastyle^{®} 300: Copolymer aus N-Vinylpyrrolidon/N-Vinylcaprolactam/N-(3-Dimethylaminopropyl)methacrylamid und 3-(Methacryloylamino)propyl-lauryl-dimethylammoniumchlorid (Aktivsubstanz 30 Gew.-% in Wasser/Ethanol, INCI-Bezeichnung: Polyquaternium-69) (ISP)
- Acudyne^{®} SCP: Copolymer aus Acrylamid und 2-Acrylamido-2-methyl-1-propansulfonat Natriumsalz (ca. 25 bis 27 Gew.-% Aktivsubstanz in Wasser, INCI-Bezeichnung: Acrylamide/Sodium Acryloyldimethyltaurate/Acrylic Acid Copolymer) (Rohm & Haas)
- Celquat^{®} L 200: quaterniertes Cellulose-Derivat (INCI-Bezeichnung: Polyquaternium-4) (National Starch)
- Gafquat^{®} 755 N PW: Dimethylaminoethylmethacrylat-Vinylpyrrolidon-Copolymer, quaterniert mit Diethylsulfat (ca. 19% Festkörper in Wasser; INCI-Bezeichnung: Polyquaternium-11) (ISP)
- Luviset^{®} Clear: Vinylpyrrolidon-Methacrylamid-Vinylimidazol-Copolymerisat (19-21% Festkörper in Wasser; INCI-Bezeichnung: VP/Methacrylamide/Vinyl Imidazole Copolymer) (BASF)
- Luviskol^{®} K85: Polyvinylpyrrolidon (ca. 20% Festkörper in Wasser; INCI-Bezeichnung: PVP) (BASF)
- Luviskol^{®} VA 64 W: Copolymer aus Vinylpyrrolidon und Vinylacetat (48-52% Aktivsubstanz in Wasser, INCI-Bezeichnung: VP/VA Copolymer) (BASF)
- Luviquat^{®} FC 370: 3-Methyl-1-vinylimidazoliumchlorid-Vinylpyrrolidon-Copolymerisat (30:70) (38-42% Festkörper in Wasser; INCI-Bezeichnung: Polyquaternium-16) (BASF)
- Luviquat^{®} Supreme: Vinylpyrrolidon-Methacrylamid-Vinylimidazol-Vinylimidazoliummethosulfat-Copolymerisat (55:29:10:6) (19-21% Festkörper in Wasser; INCI-Bezeichnung: Polyquaternium-68) (BASF)
- Styleze^{®} W-10: Copolymer aus N-Vinylpyrrolidon, N,N-Dimethylaminopropylmethacrylamid und N,N-Dimethyl-N-dodecylammoniopropylmethacrylamid chlorid (ca. 9 bis 11 % Aktivsubstanz, INCI-Bezeichnung: Polyquaternium-55) (ISP)
- Styleze^{®} CC 10: Copolymer aus N-Vinylpyrrolidon und N,N-Dimethylaminopropylmethacrylamid (ca. 9 bis 11 % Aktivsubstanz, INCI-Bezeichnung:VP/DMAPA Acrylates Copolymer) (ISP)

## Patentansprüche

1. Mittel zur temporären Verformung keratinhaltiger Fasern, insbesondere menschlichem Haar, enthaltend in einem kosmetisch akzeptablen Träger
(a) mindestens ein amphiphiles, kationisches Polymer, umfassend mindestens eine Struktureinheit der Formel (I), mindestens eine Struktureinheit der Formel (II), mindestens eine Struktureinheit der Formel (III) und mindestens eine Struktureinheit der Formel (IV), 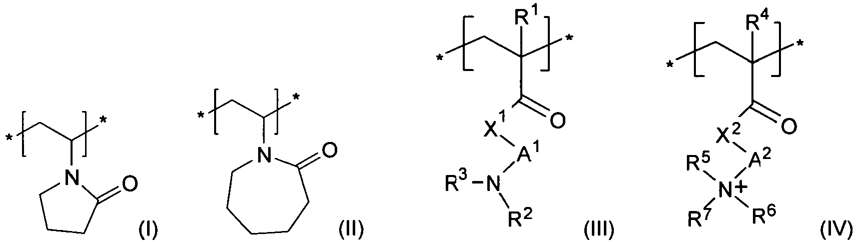 worin
R¹ und R⁴ stehen unabhängig voneinander für ein Wasserstoffatom oder eine Methylgruppe,
X¹ und X² stehen unabhängig voneinander für ein Sauerstoffatom oder eine Gruppe NH,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe und
(b) mindestens ein weiteres filmbildendes kationisches und/oder festigendes kationisches Polymer
und
(c) mindestens ein filmbildendes nichtionisches und/oder festigendes nichtionisches Polymer.

2. Mittel nach Anspruch 1, **dadurch gekennzeichnet, dass** gemäß Formel (III) beziehungsweise Formel (IV) R¹ und R⁴ jeweils eine Methylgruppe bedeuten.

3. Mittel nach einem der Ansprüche 1 oder 2, **dadurch gekennzeichnet, dass** gemäß Formel (III) beziehungsweise Formel (IV) R², R³, R⁵ und R⁶ stehen unabhängig voneinander für Methyl oder Ethyl, (besonders bevorzugt für Methyl),

4. Mittel nach einem der Ansprüche 1 bis 3, **dadurch gekennzeichnet, dass** gemäß Formel (IV) R⁷ steht für eine (C₁₀ bis C₂₄)-Alkylgruppe, insbesondere für Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

5. Mittel nach einem der Ansprüche 1 bis 4, **dadurch gekennzeichnet, dass** das darin enthaltene amphiphile, kationische Polymer mindestens eine Struktureinheit der Formel (I), mindestens eine Struktureinheit der Formel (II), mindestens eine Struktureinheit der Formel (III-8) und mindestens eine Struktureinheit der Formel (IV-8) umfasst 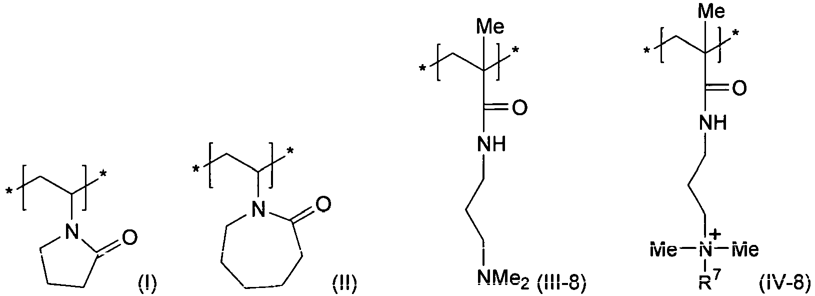 worin R⁷ steht für Octyl (Capryl), Decyl (Caprinyl), Dodecyl (Lauryl), Tetradecyl (Myristyl), Hexadecyl (Cetyl), Octadecyl (Stearyl), Eicosyl (Arachyl) oder Docosyl (Behenyl).

6. Mittel nach einem der Ansprüche 1 bis 5, **dadurch gekennzeichnet, dass** die besagten amphiphilen, kationischen Polymere in einer Menge von 0,1 Gew.-% bis 20,0 Gew.-%, insbesondere von 0,2 Gew.-% bis 10,0 Gew.-%, jeweils bezogen auf das Gewicht des Mittels, enthalten sind.

7. Mittel nach einem der Ansprüche 1 bis 6, **dadurch gekennzeichnet, dass** als filmbildendes kationisches und/oder festigendes kationisches Polymer (b) mindestens ein Polymer enthalten ist, das mindestens ein Strukturelement der Formel (M1) enthält 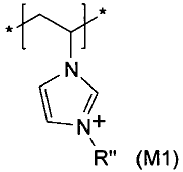 worin R" für eine (C₁ bis C₄)-Alkylgruppe, insbesondere eine Methylgruppe, steht,
und zusätzlich mindestens ein weiteres kationisches und/oder nichtionisches Strukturelement aufweist.

8. Mittel nach einem der Ansprüche 1 bis 7, **dadurch gekennzeichnet, dass** weitere filmbildende kationische und/oder festigende kationische Polymere (b) ausgewählt werden aus mindestens einem kationischen, quaternisierten Cellulose-Derivat.

9. Mittel nach einem der Ansprüche 1 bis 8, **dadurch gekennzeichnet, dass** mindestens ein zusätzliches filmbildendes kationisches und/oder festigendes kationisches Polymer (b) enthalten ist, umfassend mindestens eine Struktureinheit der Formel (I) und mindestens eine Struktureinheit der Formel (VI) und gegebenenfalls mindestens eine Struktureinheit der Formel (V) 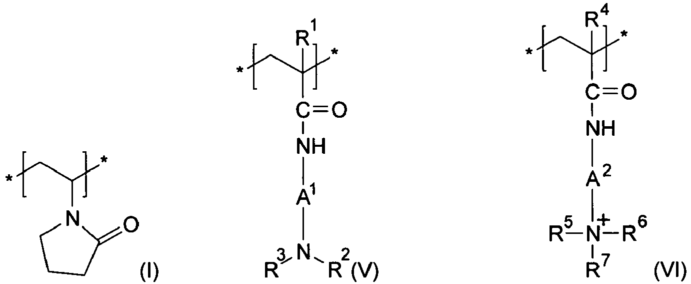 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R², R³, R⁵ und R⁶ stehen unabhängig voneinander für eine (C₁ bis C₄)-Alkylgruppe,
R⁷ steht für eine (C₈ bis C₃₀)-Alkylgruppe.

10. Mittel nach einem der Ansprüche 1 bis 9, **dadurch gekennzeichnet, dass** die filmbildenden nichtionischen und/oder festigenden nichtionischen Polymere (c) ausgewählt werden aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid.

11. Mittel nach einem der Ansprüche 1 bis 10, **dadurch gekennzeichnet, dass** die filmbildenden nichtionischen und/oder festigenden nichtionischen Polymere in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten sind.

12. Mittel nach einem der Ansprüche 1 bis 11, **dadurch gekennzeichnet, dass** zusätzlich mindestens ein Treibmittel enthalten ist.

13. Mittel nach einem der Ansprüche 1 bis 12, **dadurch gekennzeichnet, dass** es in Form eines Aerosolschaums oder Aerosolsprays vorliegt.

14. Verfahren zur Behandlung keratinhaltiger Fasern, insbesondere menschlicher Haare, worin unter Einsatz einer Abgabevorrichtung ein Mittel gemäß wenigstens eines der Ansprüche 1 bis 13 zu einem Schaum verschäumt wird und der resultierende Schaum auf die keratinhaltigen Fasern appliziert wird und worin die Fasern in Form gebracht werden und diese Form durch das besagte Mittel fixiert wird.

15. Verfahren zur Behandlung keratinhaltiger Fasern, insbesondere menschlicher Haare, worin unter Einsatz einer Abgabevorrichtung ein Mittel gemäß wenigstens eines der Ansprüche 1 bis 13 als Spray auf die keratinhaltigen Fasern appliziert wird und worin die Fasern in Form gebracht werden und diese Form durch das besagte Mittel fixiert wird.

## Claims

1. An agent for temporarily shaping keratin-containing fibers, in particular human hair, containing in a cosmetically acceptable carrier:
(a) at least one amphiphilic, cationic polymer comprising at least one structural unit of formula (I), at least one structural unit of formula (II), at least one structural unit of formula (III) and at least one structural unit of formula (IV), 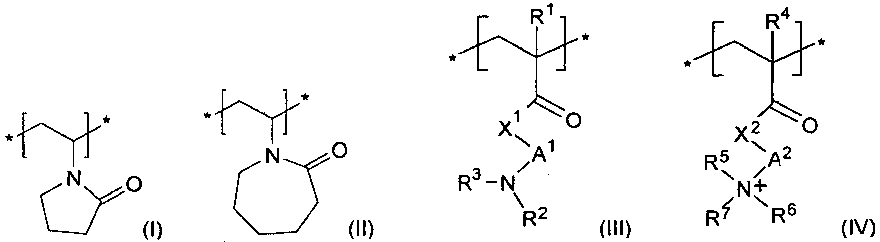 where
R¹ and R⁴ represent, independently of one another, a hydrogen atom or a methyl group,
X¹ and X² represent, independently of one another, an oxygen atom or an NH group,
A¹ and A² represent, independently of one another, an ethane-1,2-diyl, propane-1,3-diyl or butane-1,4-diyl group,
R², R³, R⁵ and R⁶ represent, independently of one another, a (C₁ to C₄) alkyl group,
R⁷ represents a (C₈ to C₃₀) alkyl group
and
(b) at least one additional film-forming cationic and/or setting cationic polymer,
and
(c) at least one film-forming non-ionic and/or setting non-ionic polymer.

2. The agent according to claim 1, **characterized in that**, in formula (III) and formula (IV), respectively, R¹ and R⁴ are a methyl group.

3. The agent according to one of claims 1 or 2, **characterized in that**, in formula (III) and formula (IV), respectively, R², R³, R⁵ and R⁶ are, independently of one another, methyl or ethyl (particularly preferably methyl).

4. The agent according to one of claims 1 to 3, **characterized in that**, in formula (IV), R⁷ represents a (C₁₀ to C₂₄) alkyl group, in particular decyl (caprinyl), dodecyl (lauryl), tetradecyl (myristyl), hexadecyl (cetyl), octadecyl (stearyl), eicosyl (arachyl) or docosyl (behenyl).

5. The agent according to one of claims 1 to 4, **characterized in that** the amphiphilic, cationic polymer contained therein comprises at least one structural unit of formula (I), at least one structural unit of formula (II), at least one structural unit of formula (III-8) and at least one structural unit of formula (IV-8), 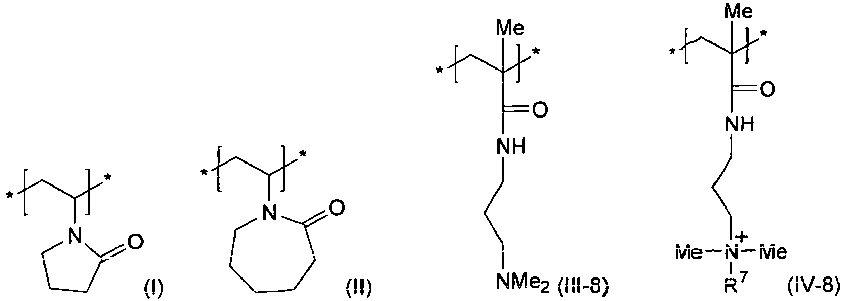 where R⁷ represents octyl (capryl), decyl (caprinyl), dodecyl (lauryl), tetradecyl (myristyl), hexadecyl (cetyl), octadecyl (stearyl), eicosyl (arachyl) or docosyl (behenyl).

6. The agent according to one of claims 1 to 5, **characterized in that** said amphiphilic, cationic polymers are contained in an amount of 0.1 wt.% to 20.0 wt.%, in particular of 0.2 wt.% to 10.0 wt.%, in each case with respect to the weight of the agent.

7. The agent according to one of claims 1 to 6, **characterized in that** at least one polymer containing at least one structural element of formula (M1) is contained as the film-forming cationic and/or setting cationic polymer (b), 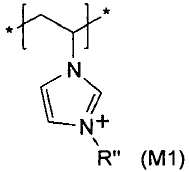
where R" represents a (C₁ to C₄) alkyl group, in particular a methyl group,
and additionally comprises at least one additional cationic and/or non-ionic structural element.

8. The agent according to one of claims 1 to 7, **characterized in that** additional film-forming cationic and/or setting cationic polymers (b) are selected from at least one cationic, quaternized cellulose derivative.

9. The agent according to one of claims 1 to 8, **characterized in that** at least one additional film-forming cationic and/or setting cationic polymer (b) is contained which comprises at least one structural unit of formula (I) and at least one structural unit of formula (VI) and optionally at least one structural unit of formula (V), 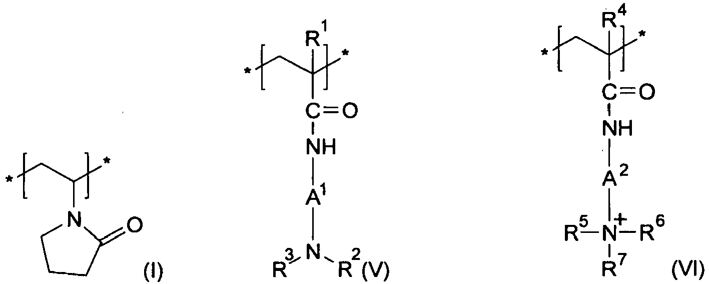 where
R¹ and R⁴ represent, independently of one other, a hydrogen atom or a methyl group,
A¹ and A² represent, independently of one another, an ethane-1,2-diyl, propane-1,3-diyl or butane-1,4-diyl group,
R², R³, R⁵ and R⁶ represent, independently of one another, a (C₁ to C₄) alkyl group,
R⁷ represents a (C₈ to C₃₀) alkyl group.

10. The agent according to one of claims 1 to 9, **characterized in that** the film-forming non-ionic and/or setting non-ionic polymer (c) is selected from
- polyvinylpyrrolidone,
- copolymers of N-vinylpyrrolidone and vinyl esters of carboxylic acids having 2 to 18 carbon atoms, in particular of N-vinylpyrrolidone and vinyl acetate,
- copolymers of N-vinylpyrrolidone and N-vinylimidazole and methacrylamide,
- copolymers of N-vinylpyrrolidone and N-vinylimidazole and acrylamide,
- copolymers of N-vinylpyrrolidone with N,N-di-(C₁ to C₄)-alkylamino-(C₂ to C₄)alkylacrylamide.

11. The agent according to one of claims 1 to 10, **characterized in that** the film-forming non-ionic and/or setting non-ionic polymers are contained in an amount of 0.1 wt.% to 20.0 wt.%, particularly preferably 0.2 wt.% to 15.0 wt.%, very particularly preferably 0.5 wt.% to 10.0 wt.%, in each case with respect to the weight of the agent according to the invention.

12. The agent according to one of claims 1 to 11, **characterized in that** at least one propellant is also contained.

13. The agent according to one of claims 1 to 12, **characterized in that** it is in the form of an aerosol foam or aerosol spray.

14. A method for treating keratin-containing fibers, in particular human hair, wherein an agent according to at least one of claims 1 to 13 is foamed to a foam using a dispensing device and the resulting foam is applied to the keratin-containing fibers, and wherein the fibers are brought into a shape and this shape is fixed by means of said agent.

15. A method for treating keratin-containing fibers, in particular human hair, wherein an agent according to at least one of claims 1 to 13 is applied as a spray to the keratin-containing fibers using a dispensing device, and wherein the fibers are brought into a shape and this shape is fixed by means of said agent.

## Revendications

1. Agents de mise en forme temporaire de fibres de kératine, en particulier de cheveux humains, contenant dans un support cosmétiquement acceptable,
(a) au moins un polymère cationique amphiphile comportant au moins une unité structurelle de la formule (I), au moins une unité structurelle de la formule (II), au moins une unité structurelle de la formule (III) et au moins une unité structurelle de la formule (IV), 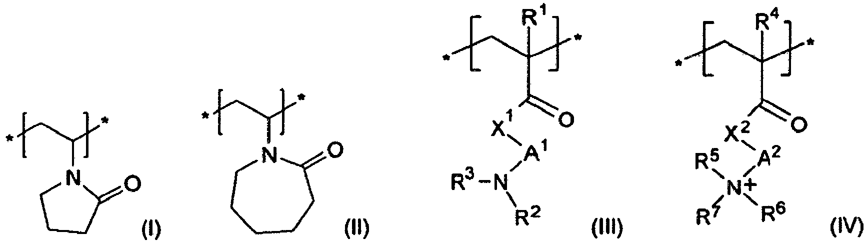 dans lesquelles
R¹ et R⁴ représentent indépendamment un atome d'hydrogène ou un groupe méthyle,
X¹ et X² représentent indépendamment un atome d'oxygène ou un groupe NH,
A¹ et A² représentent indépendamment un groupe éthane-1,2-diyle, propane-1,3-diyle ou butane-1,4-diyle,
R², R³, R⁵ et R⁶ représentent indépendamment un groupe alkyle C₁ à C₄,
R⁷ représente un groupe alkyle C₈ à C₃₀
et
(b) au moins un autre polymère cationique filmogène et/ou cationique fixant
et
(c) au moins un polymère non ionique filmogène et/ou non-ionique fixant.

2. Agent selon la revendication 1, **caractérisé en ce que**, selon la formule (III) ou la formule (IV), R¹ et R⁴ représentent chacun un groupe méthyle.

3. Agent selon l'une des revendications 1 ou 2, **caractérisé en ce que**, selon la formule (III) ou la formule (IV), R², R³, R⁵ et R⁶ sont indépendamment méthyle ou éthyle (de façon particulièrement préféré méthyle).

4. Agent selon l'une des revendications 1 à 3, **caractérisé en ce que**, selon la formule (IV), R⁷ représente un groupe alkyle en C₁₀ à C₂₄, en particulier décyle (caprinyle), dodécyle (lauryle), tétradécyle (myristyle), hexadécyle (cétyle), octadécyle (stéaryle), eicosyle (arachyle) ou docosyle (béhényle).

5. Agent selon l'une des revendications 1 à 4, **caractérisé en ce que** le polymère cationique amphiphile qu'il contient comporte au moins une unité structurelle de la formule (I), au moins une unité structurelle de la formule (II), au moins une unité structurelle de la formule (III-8) et au moins une unité structurelle de la formule (IV-8) 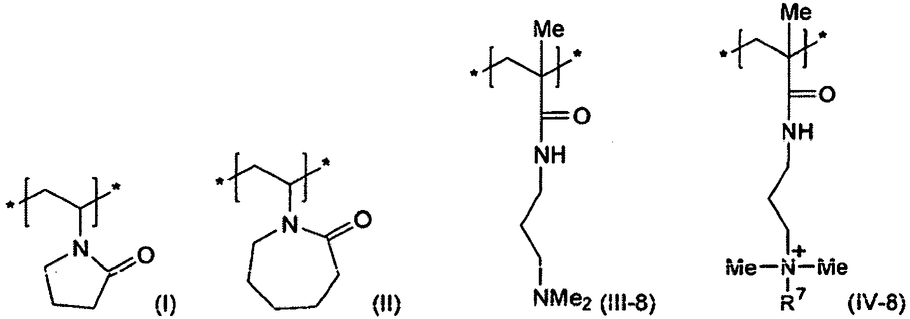 dans lesquelles R⁷ représente un groupe octyle (capryle), décyle (caprinyle), dodécyle (lauryle), tétradécyle (myristyle), hexadécyle (cétyle), octadécyle (stéaryle), eicosyle (arachyle) ou docosyle (béhényle).

6. Agent selon l'une des revendications 1 à 5, **caractérisé en ce que** lesdits polymères cationiques amphiphiles sont contenus dans une quantité de 0,1% en poids à 20,0% en poids, en particulier de 0,2% en poids à 10,0% en poids, à chaque fois par rapport au poids de l'agent.

7. Composition selon l'une des revendications 1 à 6, **caractérisé en ce qu'**il contient comme polymère (b) cationique filmogène et/ou cationique fixant au moins un polymère qui contient au moins un élément structurel de la formule (M1) 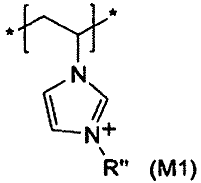 dans laquelle R" représente un groupe alkyle en C₁ à C₄, en particulier un groupe méthyle, et qui comporte en outre au moins un autre élément structurel cationique et/ou non-ionique.

8. Agent selon l'une des revendications 1 à 7, **caractérisé en ce que** d'autres polymères (b) cationiques filmogènes et/ou cationiques fixants sont choisis parmi au moins un dérivé quaternisé cationique de la cellulose.

9. Agent selon l'une des revendications 1 à 8, **caractérisé en ce qu'**il contient au moins un polymère (b) cationique filmogène et/ou cationique fixant supplémentaire, comportant au moins une unité structurelle de la formule (I) et au moins une unité structurelle de la formule (VI) et éventuellement au moins une unité structurelle de la formule (V) 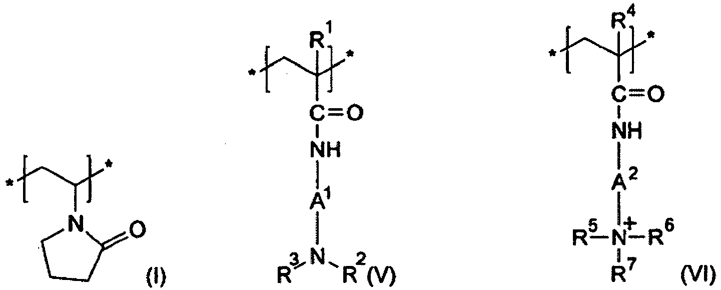 dans lesquelles
R¹ et R⁴ représentent indépendamment un atome d'hydrogène ou un groupe méthyle,
A¹ et A² représentent indépendamment un groupe éthane-1,2-diyle, propane-1,3-diyle ou butane-1,4-diyle,
R², R³, R⁵ et R⁶ représentent indépendamment un groupe alkyle C₁ à C₄,
R⁷ représente un groupe alkyle en C₈ à C₃₀.

10. Agent selon l'une des revendications 1 à 9, **caractérisé en ce que** les polymères (c) non ioniques filmogènes et/ou non ioniques fixants (C) sont choisis parmi
- la polyvinylpyrrolidone,
- les copolymères de N-vinylpyrrolidone et d'esters vinyliques d'acides carboxyliques ayant 2 à 18 atomes de carbone, en particulier de N-vinylpyrrolidone et d'acétate de vinyle,
- les copolymères de N-vinylpyrrolidone et de N-vinylimidazole et de méthacrylamide,
- les copolymères de N-vinylpyrrolidone et de N-vinylimidazole et d'acrylamide,
- les copolymères de N-vinylpyrrolidone avec du N,N-di(C₁ à C₄)-alkylamino-(C₂ à C₄)-alkylacrylamide.

11. Agent selon l'une des revendications 1 à 10, **caractérisé en ce que** les polymères non-ioniques filmogènes et/ou non-ioniques fixants sont contenus dans une quantité de 0,1 à 20,0% en poids, de façon particulièrement préférée de 0,2 à 15,0% en poids, de façon tout particulièrement préférée de 0,5 à 10,0% en poids, à chaque fois par rapport au poids de l'agent de l'invention.

12. Agent selon l'une des revendications 1 à 11, **caractérisé en ce qu'**il contient en outre au moins un agent propulseur.

13. Agent selon l'une des revendications 1 à 12, **caractérisé en ce qu'**il se présente sous la forme d'une mousse aérosol ou d'un spray aérosol.

14. Procédé de traitement de fibres de kératine, et en particulier de cheveux humains, dans lequel un agent selon au moins une des revendications 1 à 13, est transformé en mousse à l'aide d'un distributeur et la mousse résultante est appliquée sur les fibres de kératine et dans lequel les fibres sont mis forme de et cette forme est fixée par ledit agent.

15. Procédé de traitement de fibres de kératine, en particulier de cheveux humains, dans lequel l'agent selon au moins une des revendications 1 à 13 est appliqué sus forme de spray sur les fibres de kératine à l'aide d'un distributeur et dans lequel les fibres sont mises en forme et cette forme est fixée par ledit agent.
